(19) Europäisches Patentamt
European Patent Office
Office européen des brevets

(11) Numéro de publication: **0 305 298 B1**

(12) # FASCICULE DE BREVET EUROPEEN

(45) Date de publication de fascicule du brevet: **22.09.93**

(51) Int. Cl.5: **C07D 487/04**, C07D 487/14, C07D 471/14, A61K 31/55, //(C07D487/04,243:00,235:00),(C07D487/14,243:00,235:00,209:00),(C07D487/14,243:00,235:00,223:00),(C07D471/14,243:00,235:00,221:00)

(21) Numéro de dépôt: **88402166.8**

(22) Date de dépôt: **26.08.88**

(54) **Nouvelles imidazo benzodiazépines et leurs sels d'addition avec les acides, leur procédé et les intermédiaires de préparation, leur application comme médicaments et les compositions les renfermant.**

(30) Priorité: **28.08.87 GB 8720414**

(43) Date de publication de la demande:
**01.03.89 Bulletin 89/09**

(45) Mention de la délivrance du brevet:
**22.09.93 Bulletin 93/38**

(84) Etats contractants désignés:
**AT BE CH DE ES FR GR IT LI LU NL SE**

(56) Documents cités:
**EP-A- 0 027 214**

(73) Titulaire: **ROUSSEL-UCLAF**
**35, Boulevard des Invalides**
**F-75007 Paris(FR)**

(72) Inventeur: **Gardner, Colin Robert**
**Foxlea Hermitage Road**
**Cold Ash Newbury Berks(GB)**
Inventeur: **Hedgecock, Charles John Robert**
**186, High Street**
**Wootton Bassett Wiltshire SN4 7BZ(GB)**

(74) Mandataire: **Fritel, Hubert et al**
**111, route de Noisy B.P. no 9**
**F-93230 Romainville (FR)**

EP 0 305 298 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles imidazo benzodiazépines et leurs sels, ainsi que le procédé et les intermédiaires de préparation, l'application à titre de médicaments de ces nouveaux produits et les compositions les renfermant.

La demande de brevet européen EP-A-0.027.214 décrit des dérivés d'Imidazo [1,5-a] [1,4] diazépines substitués en position 3 par un groupement cyano, un groupement alcanoyle ou un groupement alcoxy carboxylique.

L'invention a pour objet de nouvelles imidazo benzodiazépines répondant à la formule générale (I) :

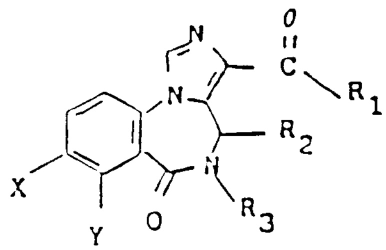

(I)

dans laquelle $R_1$ représente un radical phényle, cycloalcoyle renfermant de 4 à 6 atomes de carbone ou un groupement de formule (II) :

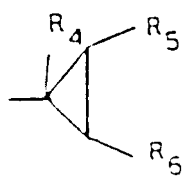

(II)

dans laquelle $R_4$ représente un atome d'hydrogène ou un radical alcoyle linéaire ou ramifié renfermant de 1 à 5 atomes de carbone, $R_5$ représente un at ome d'hydrogène ou un radical alcoyle linéaire ou ramifié renfermant de 1 à 5 atomes de carbone, $R_6$ représente un atome d'hydrogène, un radical alcoyle linéaire ou ramifié renfermant de 1 à 5 atomes de carbone, un radical phényle, un atome d'halogène, un radical alkoxycarbonyle renfermant de 2 à 5 atomes de carbone, un radical cyano, un radical amido, un radical mono- ou dialcoylamido dont les groupements alcoyles linéaires ou ramifiés renferment de 1 à 5 atomes de carbone, $R_2$ et $R_3$, identiques ou différents, représentent un atome d'hydrogène, un radical alcoyle linéaire ou ramifié, renfermant de 1 à 5 atomes de carbone, un radical cycloalcoyle renfermant de 3 à 5 atomes de carbone, ou $R_2$ et $R_3$ forment ensemble un groupement alkylène renfermant de 3 à 5 atomes de carbone, X et Y, identiques ou différents, représentent un atome d'hydrogène, un atome d'halogène, un radical alcoyle ou alcoxy renfermant de 1 à 3 atomes de carbone, un groupement nitro, azido, nitrile ou trifluorométhyle, ainsi que leurs sels d'addition avec les acides minéraux ou organiques.

Dans la formule générale (I) et dans ce qui suit :

- par radical cycloalcoyle renfermant de 4 à 6 atomes de carbone, on entend de préférence un radical cyclobutyle, cyclopentyle ou cyclohexyle ;
- par radical alcoyle renfermant de 1 à 5 atomes de carbone, on entend de préférence un radical méthyle, éthyle, propyle, isopropyle, butyle, isobutyle, terbutyle ou pentyle ;
- par atome d'halogène, on entend de préférence un atome de fluor, de chlore ou de brome ;
- par radical alkoxy-carbonyle renfermant de 2 à 5 atomes de carbone, on entend de préférence un radical méthoxy-carbonyle, éthoxy-carbonyle ou propoxy-carbonyle ;
- par radical mono ou dialcoylamido dont les radicaux alcoyles renferment de 1 à 5 atomes de carbone, on entend de préférence un radical monométhyl amido, diméthyl amido, monoéthyl amido, diéthyl amido, mono propyl amido ou dipropyl amido ;
- par radical alcoxy renfermant de 1 à 3 atomes de carbone, on entend de préférence un radical méthoxy, éthoxy, propoxy ou isopropoxy.

Les sels d'addition avec les acides minéraux ou organiques peuvent être, par exemple, les sels formés avec les acides chlorydrique, bromhydrique, iodhydrique, nitrique, sulfurique, phosphorique, propionique,

formique, benzoïque, maléïque, fumarique, succinique, tartrique, citrique, oxalique, glyoxylique, aspartique, alcanesulfoniques tel que l'acide méthanesulfonique et arylsulfoniques tel que l'acide benzènesulfonique.

Parmi les produits, objet de l'invention, on peut citer notamment les derivés répondant à la formule (I) ci-dessus, ainsi que leurs sels, caractérisés en ce que dans ladite formule (I), $R_1$ représente un groupement cyclopropyle et $R_2$, $R_3$, X et Y ont la signification déjà indiquée ; parmi ces derniers, on peut citer les dérivés répondant à la formule (I) ci-dessus ainsi que leurs sels, caractérisés en ce que dans ladite formule (I) $R_1$ représente un groupement cyclopropyle, $R_2$ et $R_3$, identiques ou différents, représentent un atome d'hydrogène ou un radical méthyle, X et Y, identiques ou différents, représentent un atome d'hydrogène, un atome de fluor ou de brome. Parmi les produits objets de l'invention, on retient tout particulièrement le produit dont le nom suit : la 3-(5,6-dihydro-5-méthyl-6-oxo-4H-imidazo [1,5a] [1,4] benzodiazépinyl)-cyclopropylméthanone.

L'invention a également pour objet un procédé de préparation des nouvelles imidazo-benzodiazépines répondant à la formule ($I_A$) :

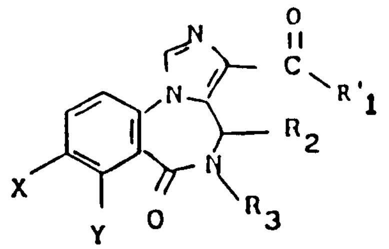

($I_A$)

dans laquelle, $R_2$, $R_3$, X et Y ont la signification déjà indiquée et $R'_1$ représente un radical phényle, cycloalcoyle renfermant de 4 à 6 atomes de carbone ou un groupement de formule II dans laquelle $R_4$ représente un atome d'hydrogène et $R_5$ et $R_6$ identiques ou différents représentent un atome d'hydrogène ou un radical alcoyle renfermant de 1 à 5 atomes de carbone, ainsi que de leurs sels, caractérisé en ce que l'on fait réagir un produit de formule (V) :

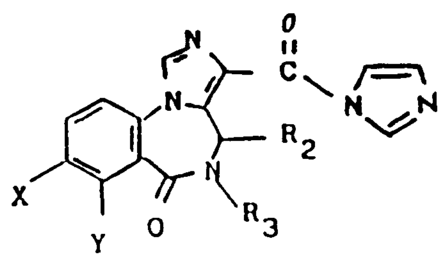

(V)

dans laquelle X, Y, $R_2$ et $R_3$ ont la signification indiquée précédemment, avec un produit de formule (VI) :

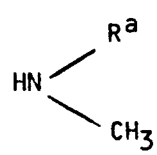

(VI)

dans laquelle $R^a$ représente un radical méthyle ou méthoxy ou un sel d'addition avec les acides, pour obtenir un produit de formule III :

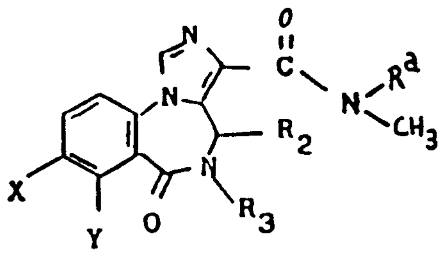

(III)

dans laquelle $R_2$, $R_3$, $R^a$, X et Y ont la signification déjà indiquée, puis fait réagir ledit produit de formule III avec un produit de formule IV :

M - $R'_1$ (IV)

dans laquelle M représente un atome de métal alcalin tel qu'un atome de lithium ou un groupe -Mg-Hal dans lequel Hal représente un atome de chlore, de brome ou d'iode et $R'_1$ a la signification déjà indiquée pour obtenir le produit de formule $I_A$ attendu que l'on peut salifier le cas échéant.

Dans des conditions préférentielles de mise en oeuvre de l'invention, le procédé de préparation ci-dessus décrit est caractérisé en ce que

- la réaction du produit de formule V avec le produit de formule VI est effectuée avantageusement au sein du diméthylformamide ;
- la réaction du produit de formule III avec le produit de formule IV est effectuée dans des conditions anhydres, dans un solvant organique tel que le tétrahydrofuranne.

Les produits de formule V peuvent être préparés par exemple selon le brevet européen N¤ 109921.

L'invention a également pour objet un procédé de préparation des nouvelles imidazobenzodiazépines de formule $I_A$ telle que définie ci-dessus ainsi que de leurs sels caractérisé en ce que l'on fait réagir un produit de formule VIII :

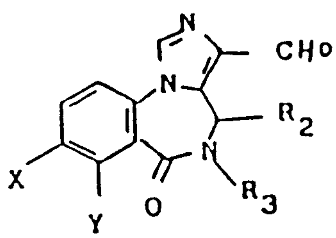

(VIII)

dans laquelle X, Y, $R_2$ et $R_3$ ont la signification déjà indiquée avec un produit de formule IV telle que définie ci-dessus pour obtenir un produit de formule (VII) :

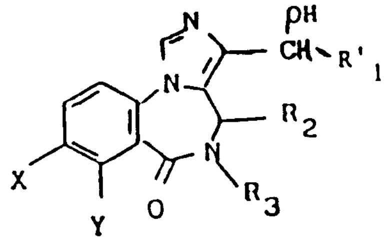

(VII)

dans laquelle $R'_1$, $R_2$, $R_3$, X et Y ont la signification déjà indiquée, puis oxyde le produit de formule (VII) ainsi obtenu, pour obtenir le produit de formule ($I_A$) attendu que l'on peut salifier le cas échéant.

Dans des conditions préférentielles de mise en oeuvre de l'invention, le procédé de préparation ci-dessus décrit est caractérisé en ce que :

- la réaction du produit de formule (VIII) avec le produit de formule (IV) est effectuée dans des conditions anhydres, dans un solvant organique tel que tétrahydrofuranne ;

- l'oxydation du produit de formule (VII) est effectuée de préférence avec le dioxyde de manganèse, l'acide nitrique, le clorure ferrique ou l'oxyde de chrome en présence de pyridine ou encore par la méthode d'Oppenauer ou enfin par déshydrogènation en présence d'un catalyseur à base de cuivre.

Les produits de formule (VIII) peuvent être préparés par exemple comme il est indiqué dans la demande de brevet européen publiée sous le N 0027214.

L'invention a aussi pour objet un procédé de préparation des nouvelles imidazo-benzodiazépines répondant à la formule ($I_B$) :

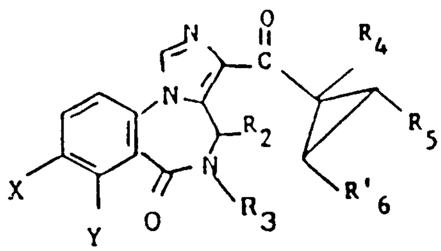

($I_B$)

dans laquelle $R_2$, $R_3$, $R_4$, $R_5$, X et Y ont la signification déjà indiquée et $R'_6$ représente un atome d'hydrogène, un atome d'halogène, un radical alcoyle renfermant de 1 à 5 atomes de carbone, un radical alkoxy carbonyle renfermant de 2 à 5 atomes de carbone, un radical phényle, cyano ou un groupement

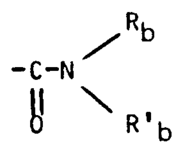

dans lequel $R_b$ et $R'_b$ identiques ou différents représentent un atome d'hydrogène ou un radical alcoyle renfermant de 1 à 5 atomes de carbone caractérisé en ce que :

- soit l'on fait réagir un produit de formule (III) telle que définie ci-dessus avec un composé de formule (X)

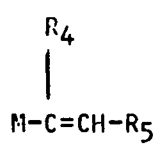

(X)

dans laquelle M, $R_4$ et $R_5$ ont la signification déjà indiquée pour obtenir un produit de formule (IX) :

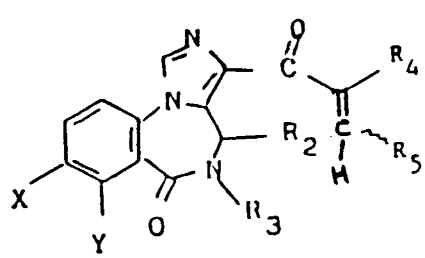

(IX)

dans laquelle $R_2$, $R_3$, $R_4$, $R_5$, X et Y ont la signification déjà indiquée,

- soit l'on fait réagir un produit de formule VIII telle que définie ci-dessus avec un composé de formule X pour obtenir un produit de formule (XI) :

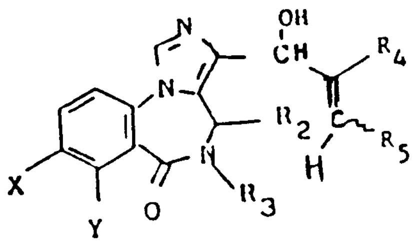

(XI)

dans laquelle $R_2$, $R_3$, $R_4$, $R_5$, X et Y ont la signification déjà indiquée, puis soumet le produit de formule (XI) ainsi obtenu, à l'action d'un agent d'oxydation pour obtenir un produit de formule (IX), produit de formule (IX) que l'on fait réagir avec un agent de cyclisation approprié pour obtenir un produit de formule ($I_B$) telle que définie ci-dessus, dans laquelle $R'_6$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, puis saponifie le cas échéant le produit obtenu dans lequel $R'_6$ représente un radical alcoxycarbonyle pour obtenir un produit de formule (XIII) :

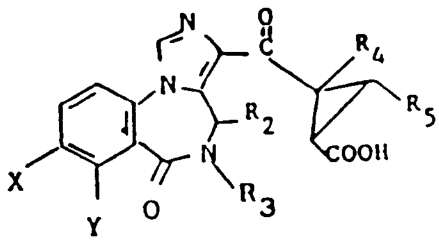

(XIII)

dans laquelle $R_2$, $R_3$, $R_4$, $R_5$, X et Y ont la signification déjà indiquée, puis fait réagir ledit produit de formule (XIII) avec une amine de formule (XII) :

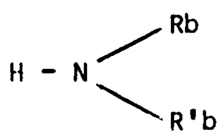

(XII)

dans laquelle Rb et R'b ont la signification déjà indiquée pour obtenir le produit correspondant de formule $I_B$ dans laquelle $R'_6$ représente un groupement de formule

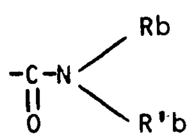

puis le cas échéant deshydrate ce dernier dans lequel Rb et R'b représentent un atome d'hydrogène pour obtenir le produit correspondant de formule $I_B$ dans laquelle $R'_6$ représente un radical cyano, et le cas échéant salifie les produits de formule $I_B$ ainsi obtenus.

Dans des conditions préférentielles de mise en oeuvre de l'invention, le procédé de préparation ci-dessus décrit est caractérisé en ce que :

- la réaction des produits de formule (III) et (VIII) avec le produit de formule (X) est effectuée dans des conditions anhydres, dans un solvant organique tel que le tétrahydrofuranne ;
- l'oxydation du produit de formule (XI) est effectuée de préférence avec le dioxyde de manganèse, l'acide nitrique, le chlorure ferrique ou l'oxyde de chrome, en présence de pyridine, ou encore par la méthode d'Oppenauer ou enfin par déshydrogénation en présence d'un catalyseur à base de cuivre ;

- l'agent de cyclisation approprié est un réactif capable d'introduire le groupement

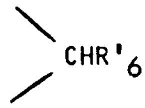

au niveau de la double liaison ;
- lorsque l'on désire obtenir un produit de formule ($I_B$) dans laquelle $R_4$ représente un radical alcoyle renfermant de 1 à 5 atomes de carbone, $R_5$ représente un atome d'hydrogène et $R'_6$ représente un atome d'hydrogène ou un radical alcoyle renfermant 1 à 5 atomes de carbone, la cyclisation est avantageusement effectuée au moyen d'un iodure de trialkyloxosulphonium en opérant au sein d'un solvant organique tel que le diméthylformamide ;
- lorsque l'on désire obtenir un produit de formule ($I_B$) dans laquelle $R_4$ représente un atome d'hydrogène et $R_5$ représente un radical alcoyle renfermant de 1 à 5 atomes de carbone et $R'_6$ représente un atome d'hydrogène ou un radical alcoyle renfermant de 1 à 5 atomes de carbone, la cyclisation est avantageusement effectuée au moyen d'un tétrafluoroborate de diméthylamino alkyl phényl oxosulphonium en opérant au sein d'un solvant organique tel que le diméthyl formamide ;
- lorsque l'on désire obtenir un produit de formule ($I_B$) dans laquelle $R_4$ représente un atome d'hydrogène et $R'_6$ représente un groupement alkoxy carbonyle ou phényle la cyclisation est avantageusement effectuée au moyen d'un acétate de diméthylsulfuranylidène ou d'un anion benzyldiméthylsulphonium en opérant au sein d'un solvant organique tel que le chloroforme ;
- lorsque l'on désire obtenir un produit de formule ($I_B$) dans laquelle $R_4$ représente un atome d'hydrogène et $R'_6$ représente un atome d'halogène, la cyclisation est avantageusement effectuée au moyen d'un halogéno méthylylide de diméthyl amino phényl oxosulphonium en opérant au sein d'un solvant organique tel que le diméthyl formamide ;
- la saponification du produit de formule ($I_B$) dans laquelle $R'_6$ représente un radical alkoxycarbonyle est effectuée de préférence au moyen d'un hydroxyde alcalin tel que l'hydroxyde de sodium ;
- la réaction du produit de formule ($I_B$) avec l'amine de formule (XII) est effectuée de préférence au sein d'un solvant organique anhydre en présence de carbonyldiimidazole ;
- la déshydration du produit de formule ($I_B$) est effectuée de préférence au moyen d'un anhydride d'acide fort tel que l'anhydride d'acide trifluoroacétique au sein d'un solvant organique tel que le dichlorométhane.

Les produits de formule (I) présentent un caractère basique.
On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques, on note en particulier de faibles propriétés agonistes inverses pour certains, et tous présentent une plus longue durée d'action en comparaison d'autres imidazobenzodiazépines similaires.

Certains produits présentent, en outre, des propriétés tranquilisantes.

Ces propriétés sont illustrées plus loin dans la partie expérimentale.

Ces propriétés justifient l'utilisation des nouvelles imidazobenzodiazépines de formule (I) ainsi que leurs sels pharmaceutiquement acceptables, à titre de médicaments.

La présente invention a aussi également pour objet l'application à titre de médicaments des nouvelles imidazo-benzodiazépines telles que définies par la formule générale (I) ainsi que de leurs sels d'addition avec les acides pharmaceutiquement acceptables.

Parmi les médicaments, objet de l'invention, on retient notamment les médicaments carractérisés en ce qu'ils sont constitués par les nouvelles imidazobenzodiazépines répondant à la formule I dans laquelle $R_1$ représente un groupement cyclopropyle, $R_2$, $R_3$, X et Y ayant la signification déjà indiquée ainsi que leurs sels d'addition avec les acides pharmaceutiquement acceptables.

Parmi les médicaments, objet de l'invention, on retient tout particulièrement ceux répondant à la formule (I), caractérisé en ce que dans ladite formule (I) $R_1$ représente un groupement cyclopropyle, $R_2$ et $R_3$, identiques ou différents, représentent un atome d'hydrogène ou un radical méthyle, X et Y, identiques ou différents, représentent un atome d'hydrogène un atome de fluor ou de brome ainsi que leurs sels d'addition avec les acides pharmaceutiquement acceptables.

Parmi les médicaments préférés de l'invention, on retient tout particulièrement : la 3-(5,6-dihydro-5-méthyl-6-oxo-4H-imidazo [1,5a] [1,4] benzodiazépinyl)-cyclopropylméthanone, ainsi que ses sels pharmaceutiquement acceptables.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquilisant mineur dans le traitement de certaines agitations ou irritabilité et dans certaines formes d'épilepsie.

La dose usuelle variable selon le produit utilisé, le sujet traité et l'affection en cause peut être par exemple, de 0,1 mg à 200 mg par jour, par voie orale.

L'invention à également pour objet les compositions pharmaceutiques qui renferment au moins un dérivé précité ou l'un de ses sels d'addition avec les acides pharmaceutiquement acceptable à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet, à titre de produits nouveaux et notamment d'intermédiaires nécessaires à la préparation des produits de formule (I) :

- Les produits de formule (VII) :

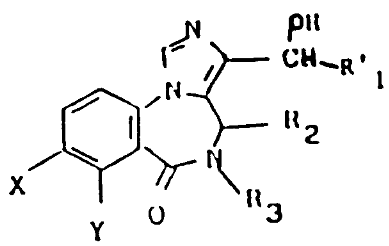

(VII)

dans laquelle $R'_1$, $R_2$, $R_3$, X et Y ont la signification déjà indiquée.

- Les produits de formule (XI) :

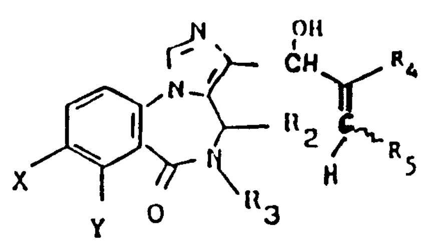

(XI)

dans laquelle $R_2$, $R_3$, $R_4$, $R_5$, X et Y ont la signification déjà indiquée.

- Les produits de formule (IX) :

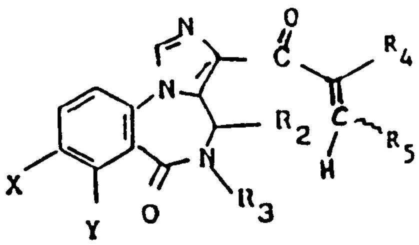

(IX)

dans laquelle $R_2$, $R_3$, $R_4$, $R_5$, ont la signification déjà indiquée.

- Les produits de formule (III) :

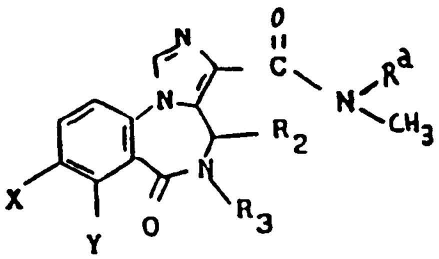

(III)

dans laquelle $R_2$, $R_3$, $R^a$, X et Y ont la signification déjà indiquée.

- Les produits de formule (XIII) :

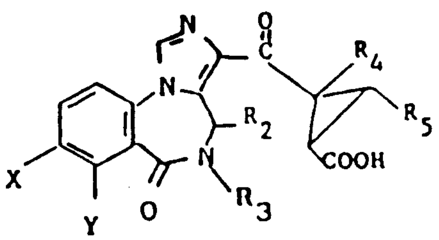

(XIII)

dans laquelle $R_2$, $R_3$, $R_4$, $R_5$, X et Y ont la signification déjà indiquée.

Il va être donné maintenant des exemples de mise en oeuvre de l'invention.

**Exemple 1 : 3-(5,6-dihydro-5-méthyl-6-oxo 4H-imidazo [1,5a] [1,4] benzodiazépinyl)-cyclopropylméthanone.**

Stade A : 3-(5,6-dihydro-5-méthyl-6-oxo-4H-imidazo[1,5a] [1,4] benzodiazépinyl)-cyclopropylméthanol.

A une solution de 1,82 g de 5,6-dihydro-5-méthyl-6-oxo-4H-imidazo[1,5a] [1,4] benzodiazépine-3-carboxaldéhyde dans 50 $cm^3$ de tétrahydrofuranne on ajoute, à température ambiante 22,7 $cm^3$ d'une solution 0,5 M de magnésien de bromure de cyclopropyle. Après 30 minutes de reflux on ajoute de nouveau 11,4 $cm^3$ de la solution de magnésien puis on agite pendant 18 heures à température ambiante. On verse la suspension dans 100 $cm^3$ d'eau et glace, filtre puis évapore le solvant et extrait avec du chloroforme. On évapore à sec sous pression réduite et chromatographie le résidu (2,5 g) sur silice, éluant chloroforme-méthanol (96-4). On obtient 1,47 g de produit attendu F = 126-128¤C cristallisé du mélange acétate d'éthyle-éther de pétrole (Eb. 60-80¤C).

Spectre RMN ($CDCl_3$) 8.05(d.d) ; 7.86(s) ; 7.61(d.t) ; 7.49(d.t) ; 7.40(d.d) 4.30-4.68(br,2H) ; 4.19(d.d) ; 3.24-(S,MeN) ; 1.39(m) ; 0.68 et 0.50 (2xm,2x2H).

Spectre IR (KBr) 3360 br ; 1640 ; 1630 ; 1495 ; 1395 ; 1225 ; 1033 ; 942 ; 760 $cm^{-1}$.

Stade B : 3-(5,6-dihydro-5-methyl-6-oxo-4H-imidazo[1,5a] [1,4] benzodiazépinyl)-cyclopropylméthanone.

On agite pendant 1 heure 1,87 g de 3-(5,6-dihydro-5-méthyl-6-oxo-4H-imidazo[1,5a] [1,4] benzodiazépinyl)-cyclopropylméthanol dans 120 cm$^3$ de chlorure de méthylène avec 5,75 g d'oxyde de manganèse ajoute 5,75 g d'oxyde de manganèse supplémentaire, agite 2 heures, filtre et évapore à sec sous pression réduite. On chromatographie le résidu sur silice éluant chloroforme. On obtient, après recristallisation dans l'acétate d'éthyle, 1,27 g de produit attendu F = 191-193¤C.
Spectre RMN ($CDCl_3$) 8.05(d.d) ; 7.88(s) ; 7.63(d.t) ; 7.53(d.t) ; 7.41(d.d) 5.28 et 4.33(2xbr,2H) ; 3.23(s,3H) ; 3.18(m) ; 1.25(br,2H) et 1.08(br,2H).
Spectre IR (KBr) 3100 ; 1735 ; 1565 ; 1495 ; 1390 ; 1250 ; 1230 ; 755 cm$^{-1}$

| Analyse | $C_{16}H_{15}N_3O_2$ | Calculé<br>Trouvé | C% | 68.31<br>68.38 | H% | 5.37<br>5.43 | N% | 14.94.<br>14.94. |
|---|---|---|---|---|---|---|---|---|

**Exemple 2 : (8-chloro-5,6-dihydro-5-méthyl-6-oxo-4H-imidazo[1,5-a] [1,4] benzodiazépin-3-yl) cyclopropylméthanone.**

Stade A: N-méthyl carbohydroxamate de (8-chloro-5,6-dihydro-5-méthyl-6-oxo-4H-imidazo[1,5-a] [1,4] benzodiazépin-3-yl (PREPARATION A).

A une solution de 4,26 g d'acide 8-chloro-5,6-dihydro-5-méthyl-6-oxo-4H-imidazo[1,5-a] [1,4]-benzodiazepine-3-carboxylique dans 70 cm$^3$ de diméthylformamide on ajoute 7,12 g de 1,1 carbonyl diimidazole, agite pendant 3 heures à 50¤C, refroidit à 0¤C et essore ; on obtient 3,79 g d'imidazolure intermédiaire que l'on agite à 60¤C avec 75 cm$^3$ de diméthyl formamide et 3,25 g de chlorhydrate de diméthyl hydroxylamine. Après 1 heure 30 on refroidit et verse dans 300 cm$^3$ d'eau ; on extrait à l'acétate d'éthyle et concentre sous pression réduite. On obtient 3,15 g de produit attendu.

Stade B : (8-chloro-5,6-dihydro-5-méthyl-6-oxo-4H-imidazo[1,5-a] [1,4] benzodiazepin-3-yl) cyclopropylméthanone.

A 20 cm$^3$ d'une solution de magnésien de bromure de cyclopropyle dans le tétrahydrofuranne on ajoute une solution de produit obtenu au stade A dans 20 cm$^3$ de tétrahydrofuranne. Après 1 heure 30 d'agitation à température ambiante le milieu est décomposé dans une solution de chlorure d'ammonium et on extrait au chloroforme. On sèche, évapore sous pression réduite et chromatographie le résidu sur silice éluant chlorure de méthylène-acétate d'éthyle. On obtient 704 mg de produit attendu cristallisé de l'acétate d'éthyle F = 230-232¤C.

Les dérivés N-méthylcarbohydroxamate suivants ont été préparés de manière identique au stade A.

Préparation B

(5,6-dihydro-5-méthyl-6-oxo-4H-imidazo[1,5-a] [1,4]-benzodiazepin-3-yl)-N-méthyl carbohydroxamate.

Préparation C

(5,6-dihydro-8-fluoro-5-méthyl-6-oxo-4H-imidazo[1,5-a] [1,4]-benzodiazepin-3-yl)-N-méthyl carbohydroxamate.

Préparation D

(8-bromo-5,6-dihydro-5-méthyl-6-oxo-4H-imidazo[1,5-a] [1,4]-benzodiazepin-3-yl)-N-méthyl carbohydroxamate.

Les dérivés cyclopropylméthanone suivants ont été préparés comme à l'exemple 2.

**Exemple 3 : (5,6-dihydro-8-fluoro-5-méthyl-6-oxo-4H-imidazo[1,5-a] [1,4] benzodiazepin-3-yl) cyclopropylméthanone.**

**Exemple 4 : (8-bromo-5,6-dihydro-5-méthyl-6-oxo-4H-imidazo[1,5-a] [1,4] benzodiazepin-3-yl) cyclopropylméthanone.**

Les dérivés phényl méthanone suivants ont été préparés comme à l'exemple 2 en utilisant le magnésien du bromure de phényl au lieu de magnésien de bromure de cyclopropyle.

**Exemple 5 : (5,6-dihydro-5-méthyl-6-oxo-4H-imidazo[1,5-a][1,4] benzodiazepin-3-yl) phénylméthanone.**

**Exemple 6 : (5,6-dihydro-8-fluoro-5-méthyl-6-oxo-4H-imidazo[1,5-a][1,4] benzodiazepin-3-yl) phénylméthanone.**

**Exemple 7 : (8-chloro-5,6-dihydro-5-méthyl-6-oxo-4H-imidazo[1,5-a][1,4] benzodiazepin-3-yl) phénylméthanone.**

**Exemple 8 : (8-bromo-5,6-dihydro-5-méthyl-6-oxo-4H-imidazo[1,5-a][1,4] benzodiazepin-3-yl) phénylméthanone.**

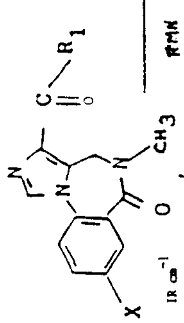

| EX OU Prep. | | $R_1$-CO- | X | Rend.‡ | F° | IR $cm^{-1}$ | RMN | Formule | C Calc Trouvé | H | N | X |
|---|---|---|---|---|---|---|---|---|---|---|---|---|
| 1 | | 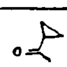 | H | 47%[A] | 191-193 EtOAC | 3100,1641,1565,1495, 1390,1230,755 | 7.41-8.05 (aromatiq,5H); 5.28 & 4.33 (br,2H); 3.23 (s,3H); 3.18 (m,1H) 1.25 (m,2H); 1.08 (m,2H) | $C_{16}H_{15}N_3O_2$ 281.32 | 68.31<br>68.38 | 5.37<br>5.43 | 14.94<br>14.94 | - |
| 3 | | " | F | 58%[B] | 185-187 | | | $C_{16}H_{14}FN_3O_2$ 299.31 | 64.20<br>63.80 | 4.72<br>4.81 | 14.03<br>13.79 | 6.35<br>6.21 |
| 2 | | " | Cl | 49%[B] | 230-232 | | 7.40-8.05 (aromatiq,4H); 5.33 & 4.35 (br,2H); 3.23 (s,3H); 3.18 (m,1H); 1.23 (m,2H); 1.09 (m,2H) | $C_{16}H_{14}ClN_3O_2$ 315.75 | 60.86 | 4.48 | 13.30 | 11.23 |
| 4 | | " | Br | 58%[B] | 228-230 | 3100,1637,1490,1392, 1248,1218,938 | 7.28-8.20 (aromatiq,4H); 5.32 & 4.30 (br,2H); 3.24 (s,3H); 3.20 (m,1H); 1.25 (m,2H); 1.11 (m,2H) | $C_{16}H_{14}BrN_3O_2$ 360.218 | 53.35 | 3.92 | 11.67 | 22.18 |
| B | | 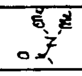 | H | | | | 7.43-8.08 (aromatiq,5H); 4.33 & 5.20 (br,2H); 3.93 (s,3H); 3.58 (s,3H); 3.30 (s,3H) | | | | | - |
| C | | " | F | 67%[C] | 176-177 | | | $C_{15}H_{15}FN_4O_3$ 318.31 | 56.60<br>56.48 | 4.76<br>4.82 | 17.59<br>17.46 | 5.97<br>5.96 |
| A | | " | Cl | 85%[C] | 160-161 | | | $C_{15}H_{15}ClN_4O_3$ 334.75 | 53.82<br>53.61 | 4.53<br>4.57 | 16.73<br>16.69 | 10.59 |
| D | | " | Br | 76%[C] | 179-180 | 3110,1635,1580, 1496,1386,1229,942 | 7.31-8.19 (aromatiq,4H); 5.20 & 4.30 (br,2H); 3.92 (s,3H); 3.57 (s,3H); 3.28 (s,3H) | $C_{15}H_{15}BrN_4O_3$ 379.221 | 47.51<br>47.51 | 3.99<br>4.00 | 14.77<br>14.67 | 21.07<br>21.30 |
| 5 | | 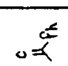 | H | | | | | | | | | - |
| 6 | | " | F | 69%[B] | | | | $C_{19}H_{14}FN_3O_2$ 335.33 | 68.05 | 4.22 | 12.52 | 5.67 |
| 7 | | " | Cl | 82%[B] | | | | $C_{19}H_{14}ClN_3O_2$ 351.78 | 64.87<br>64.77 | 4.02<br>4.13 | 11.94<br>11.84 | 10.08<br>10.22 |
| 8 | | " | Br | 41%[B] | 220-223 | 3120,1630,1542, 1494,1363,1285,902 | 7.30-8.28 (aromati ,9H); 5.35 & 4.43 (b,2H); 3.30 (s,3H) | $C_{19}H_{14}BrN_3O_2$ 396.25 | 57.59<br>57.68 | 3.56<br>3.72 | 10.60<br>10.40 | 20.17<br>19.97 |

A= de l'aldehyde B= de l'hydroxamate C= de l'imidazole

**Exemple 9 :**

On a préparé des comprimés répondant à la formulation suivante :

| | |
|---|---|
| - composé de l'exemple 1<br>- excipient q.s.q. un comprimé terminé à | 20 mg<br>150 mg |

(composition de l'excipient : lactose, amidon, talc, stéarate de magnésium).

Activité pharmacologique

Les composés de l'invention sont des agents qui ont une interaction avec les récepteurs des benzodiazépines dans le cerveau, certains d'entre eux peuvent être utiles pour le traitement de l'obésité ou des troubles de la cognition et certains d'entre eux peuvent être utiles comme tranquillisants légers. Le screening pour les liaisons de récepteurs de benzodiazépine (BRB) a été conduit selon la méthode décrite dans le brevet GB 2128929.

a) La capacité des composés a induire des contractions dans le muscle hyoidal de rats a été étudié selon la méthode de V.W. James et C.R. Gardner (Eur. J. Pharmacol (1985), 113, 233).

b) Potentialisation des convulsions induites par l'injection sous-cutanée de Leptazol à des souris $CD_1$.

On choisit une dose de Leptazol qui produit 10-20 % de convulsions chez des souris $CD_1$ non traitées. Les produits actifs augmentent le pourcentage des convulsions et la $DE_{50}$ est calculée par la méthode de Lichfield et Wilcoxon (J. Pharmacol. Exp. Ther. (1949) 96, 99).

| Composé | BRB(nM) | Contraction Hyoidale (mg/kg) | Convulsions par leptazol $DE_{50}$ (mg/kg) |
|---|---|---|---|
| 1 | 95 | 20ip (+) | 1-2ip |
| 2 | 64 | 50ip + + | Non actif |
| 3 | 560 | | |
| 4 | 354 | | |
| 6 | >10.000 | | |
| 10 | 10.000 | | |
| 11 | 3.500 | | |
| 12 | 10.000 | | |

**Revendications**

**Revendications pour les Etats contractants suivants : AT, BE, CH, DE, FR, IT, LI, LU, NL, SE**

**1.** Nouvelles imidazo benzodiazépines répondant à la formule générale (I) :

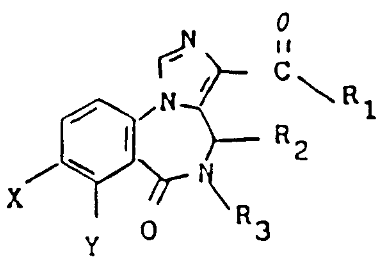

(I)

dans laquelle $R_1$ représente un radical phényle, cycloalcoyle renfermant de 4 à 6 atomes de carbone ou un groupement de formule (II) :

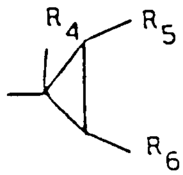

(II)

dans laquelle $R_4$ représente un atone d'hydrogène ou un radical alcoyle linéaire ou ramifié renfermant de 1 à 5 atomes de carbone, $R_5$ représente un atome d'hydrogène ou un radical alcoyle linéaire ou ramifié renfermant de 1 à 5 atomes de carbone, $R_6$ représente un atome d'hydrogène, un radical alcoyle linéaire ou ramifié renfermant de 1 à 5 atomes de carbone, un radical phényle, un atome d'halogène, un radical alkoxycarbonyle renfermant de 2 à 5 atomes de carbone, un radical cyano, un radical amido, un radical mono- ou dialcoylamido dont les groupements alcoyles linéaires ou ramifiés renferment de 1 à 5 atomes de carbone, $R_2$ et $R_3$, identiques ou différents,
représentent un atome d'hydrogène, un radical alcoyle linéaire ou ramifié, renfermant ce 1 à 5 atomes de carbone, un radical cycloalcoyle renfermant de 3 à 5 atomes de carbone, ou $R_2$ et $R_3$ forment ensemble un groupement alkylène renfermant de 3 à 5 atomes de carbone, X et Y, identiques ou différents, représentent un atome d'hydrogène, un atome d'halogène, un radical alcoyle ou alcoxy renfermant de 1 à 3 atomes de carbone, un groupement nitro, azido, nitrile ou trifluorométhyle, ainsi que leurs sels d'addition avec les acides minéraux ou organiques.

**2.** Nouvelles imidazo-benzodiazépines telles que définies par la formule (I) de la revendication 1, caractérisées en ce que dans ladite formule (I), $R_1$, représente un groupement cyclopropyle et $R_2$, $R_3$, X et Y ont la signification déjà indiquée, ainsi que leurs sels d'addition avec les acides minéraux organiques.

**3.** Nouvelles imidazo-benzodiazépines selon la revendication 1 ou 2 caractérisées en ce que dans ladite formule (I), $R_1$ représente un groupement cyclopropyle, $R_2$ et $R_3$, identiques ou différents, représentent un atome d'hydrogène, un radical méthyle, X et Y identiques ou différents, représentent un atome d'hydrogène, un atome de fluor ou de brome ainsi que leurs sels d'addition avec les acides minéraux ou organiques.

**4.** La 3-(5,6-dihydro-5-méthyl-6-oxo-4H-imidazo [1,5a] [1,4] benzodiazépinyl)-cyclopropylméthanone.

**5.** Procédé de préparation des nouvelles imidazobenzodiazépines selon la revendication 1 répondant à la formule ($I_A$) :

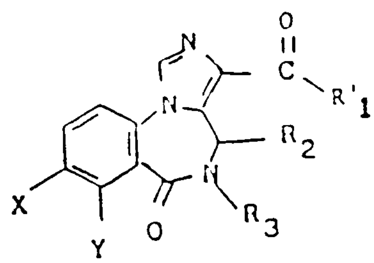

($I_A$)

dans laquelle, $R_2$, $R_3$, X et Y ont la signification déjà indiquée et $R'_1$ représente un radical phényle, cycloalcoyle renfermant de 4 à 6 atomes de carbone ou un groupement de formule II dans laquelle $R_4$ représente un atome d'hydrogène et $R_5$ et $R_6$ identiques ou différents représentent un atome d'hydrogène ou un radical alcoyle renfermant de 1 à 5 atomes de carbone, ainsi que de leurs sels, caractérisé en ce que :

- soit l'on fait réagir un produit de formule (V) :

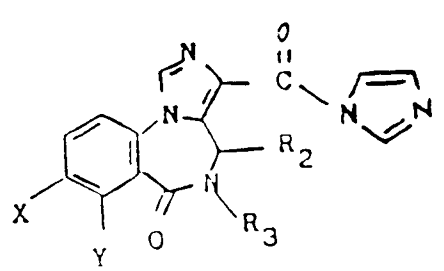

(V)

dans laquelle X, Y, $R_2$ et $R_3$ ont la signification indiquée précédemment, avec un produit de

formule (VI) :

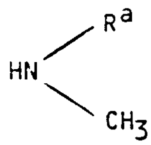

(VI)

dans laquelle $R^a$ représente un radical méthyle ou méthoxy ou un sel d'addition avec les acides, pour obtenir un produit de formule III :

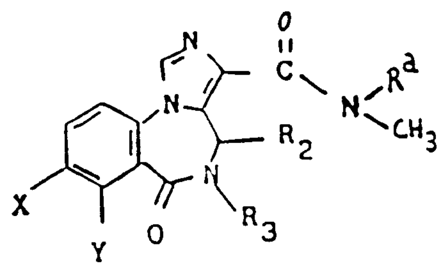

(III)

dans laquelle $R_2$, $R_3$, $R^a$, X et Y ont la signification déjà indiquée, puis fait réagir ledit produit de formule III avec un produit de formule IV :

M - $R'_1$ (IV)

dans laquelle M représente un atome de métal alcalin tel qu'un atome de lithium ou un groupe -Mg-Hal dans lequel Hal représente un atome de chlore, de brome ou d'iode et $R'_1$ a la signification déjà indiquée pour obtenir le produit de formule $I_A$ attendu que l'on peut salifier le cas échéant,

- soit l'on fait réagir un produit de formule VIII :

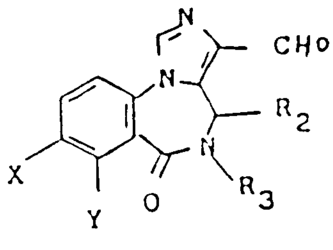

(VIII)

dans laquelle X, Y, $R_2$ et $R_3$ ont la signification déjà indiquée avec un produit de formule IV telle que définie ci-dessus pour obtenir un produit de formule (VII) :

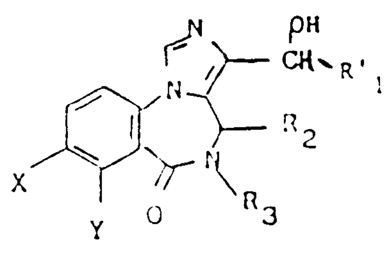

(VII)

dans laquelle $R'_1$, $R_2$, $R_3$, X et Y ont la signification déjà indiquée, puis oxyde le produit de formule (VII) ainsi obtenu, pour obtenir le produit de formule ($I_A$) attendu que l'on peut salifier le

cas échéant.

**6.** Procédé de préparation selon la revendication 5, caractérisé en ce que :
- la réaction du produit de formule V avec le produit de formule VI est effectuée avantageusement au sein du diméthylformamide ;
- la réaction du produit de formule III ou VIII avec le produit de formule IV est effectuée dans des conditions anhydres, dans un solvant organique tel que tétrahydrofuranne ;
- l'oxydation du produit de formule VII est effectuée de préférence avec le dioxyde de manganèse, l' acide nitrique, le clorure ferrique ou l'oxyde de chrome en présence de pyridine ou encore par la méthode d'Oppenauer ou enfin par déshydrogénation en présence d'un catalyseur à base de cuivre.

**7.** Procédé de préparation des nouvelles imidazobenzodiazépines telles que définies dans la formule (I) de la revendication 1 répondant à la formule $I_B$ :

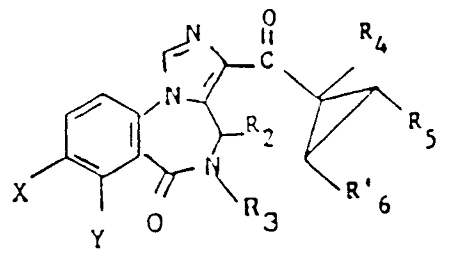

$(I_B)$

dans laquelle $R_2$, $R_3$, $R_4$, $R_5$, X et Y ont la signification déjà indiquée et $R'_6$ représente un atome d'hydrogène, un atome d'halogène, un radical alcoyle renfermant de 1 à 5 atomes de carbone, un radical alkoxy carbonyle renfermant de 2 à 5 atomes de carbone, un radical phényle, cyano ou un groupement

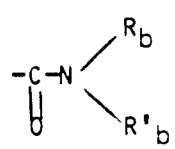

dans lequel $R_b$ et $R'_b$ identiques ou différents représentent un atome d'hydrogène ou un radical alcoyle renfermant de 1 à 5 atomes de carbone caractérisé en ce que :
- soit l'on fait réagir un produit de formule (III) telle que définie ci-dessus avec un composé de formule (X)

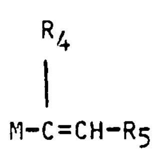

(X)

dans laquelle M, $R_4$ et $R_5$ ont la signification déjà indiquée pour obtenir un produit de formule (IX) :

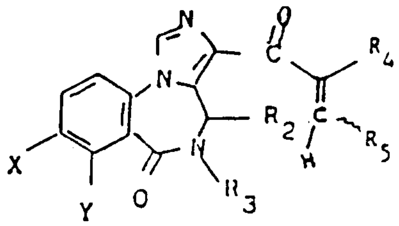

(IX)

dans laquelle $R_2$, $R_3$, $R_4$, $R_5$, X et Y ont la signification déjà indiquée,

- soit l'on fait réagir un produit de formule VIII telle que définie ci-dessus avec un composé de formule X pour obtenir un produit de formule (XI) :

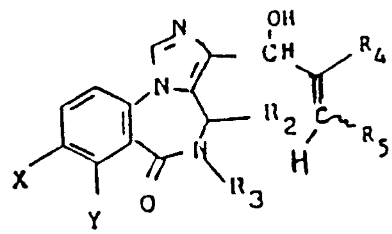

(XI)

dans laquelle $R_2$, $R_3$, $R_4$, $R_5$, X et Y ont la signification déjà indiquée, puis soumet le produit de formule (XI) ainsi obtenu, à l'action d'un agent d'oxydation pour obtenir un produit de formule (IX), produit de formule (IX) que l'on fait réagir avec un agent de cyclisation approprié pour obtenir un produit de formule ($I_B$) telle que définie ci-dessus, dans laquelle $R'_6$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, puis saponifie le cas échéant le produit obtenu dans lequel $R'_6$ représente un radical alcoxycarbonyle pour obtenir un produit de formule (XIII) :

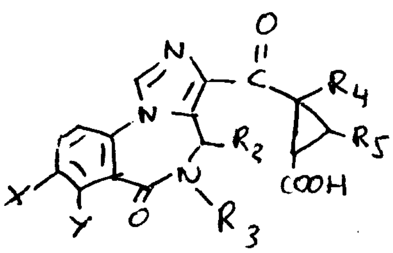

(XIII)

dans laquelle $R_2$, $R_3$, $R_4$, $R_5$, X et Y ont la signification déjà indiquée, puis fait réagir ledit produit de formule (XIII) avec une amine de formule (XII) :

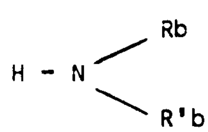

(XII)

dans laquelle Rb et R'b ont la signification déjà indiquée pour obtenir le produit correspondant de formule $I_B$ dans laquelle $R'_6$ représente un groupement de formule

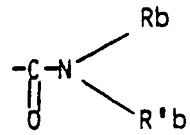

puis le cas échéant déshydrate ce dernier dans lequel Rb et R'b représentent un atome d'hydrogène pour obtenir le produit correspondant de formule $I_B$ dans laquelle $R'_6$ représente un radical cyano, et le cas echéant salifie les produits de formule $I_B$ ainsi obtenus.

**8.** Procédé de préparation selon la revendication 7, caractérisé en ce que la réaction des produits de formules (III) et (VIII) avec le produit de formule (X) est effectuée dans des conditions anhydres, dans un solvant organique tel que le tétrahydrofuranne.

**9.** Procédé de préparation selon la revendication 7, caractérisé en ce que l'oxydation du produit de formule (XI) est effectuée de préférence avec le dioxyde de manganèse, l'acide nitrique, le chlorure ferrique ou l'oxyde de chrome, en présence de pyridine, ou encore par la méthode d'Oppenauer ou enfin par déshydrogénation en présence d'un catalyseur à base de cuivre.

**10.** Procédé de préparation selon la revendication 7, caractérisé en ce que :

- l'agent de cyclisation approprié est un réactif capable d'introduire le groupement

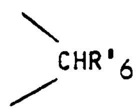

au niveau de la double liaison ;
- lorsque l'on désire obtenir un produit de formule ($I_B$) dans laquelle $R_4$ représente un radical alcoyle renfermant de 1 à 5 atomes de carbone, $R_5$ représente un atome d'hydrogène et $R'_6$ représente un atome d'hydrogène ou un radical alcoyle renfermant 1 à 5 atomes de carbone, la cyclisation est avant ageusement effectuée au moyen d'un iodure de trialkyloxosulphonium en opérant au sein d'un solvant organique tel que le diméthylformamide ;
- lorsque l'on désire obtenir un produit de formule ($I_B$) dans laquelle $R_4$ représente un atome d'hydrogène et $R_5$ représente un radical alcoyle renfermant de 1 à 5 atomes de carbone et $R'_6$ représente un atome d'hydrogène ou un radical alcoyle renfermant de 1 à 5 atomes de carbone, la cyclisation est avant ageusement effectuée au moyen d'un tétrafluoroborate de diméthylamino alkyl phényl oxosulphonium en opérant au sein d'un solvant organique tel que le diméthyl formamide ;
- lorsque l'on désire obtenir un produit de formule ($I_B$) dans laquelle $R_4$ représente un atome d'hydrogène et $R'_6$ représente un groupement alkoxy carbonyle ou phényle la cyclisation est avantageusement effectuée au moyen d'un acétate de diméthylsulfuranylidène ou d'un anion benzyldiméthylsulphonium en opérant au sein d'un solvant organique tel que le chloroforme ;
- lorsque l'on désire obtenir un produit de formule ($I_B$) dans laquelle $R_4$ représente un atome d'hydrogène et R'6 représente un atome d'halogène, la
- cyclisation est avantageusement effectuée au moyen d'un halogéno méthylylide de diméthyl amino phényl oxosulphonium en opérant au sein d'un solvant organique tel que le diméthyl formamide ;

**11.** Procédé de préparation selon la revendication 7, caractérisé en ce que :

- la saponification ou produit de formule ($I_B$) dans laquelle $R'_6$ représente un radical alkoxycarbonyle est effectuée de préférence au moyen d'un hydroxyde alcalin tel que l'hydroxyde de sodium ;
- la réaction du produit de formule ($I_B$) avec l'amine de formule (XII) est effectuée de préférence au sein d'un solvant organique anhydre en présence de carbonyldiimidazole ;
- la déshydratation du produit de formule ($I_B$) est effectuée de préférence au moyen d'un anhydride d'acide fort tel que l'anhydride d'acide trifluoroacétique au sein d'un solvant organique tel que le dichlorométhane.

**12.** Médicaments, caractérisés en ce qu'ils sont constitués par les nouvelles imidazobenzodiazépines telles que définies par la formule (I) de la revendication 1 ainsi que par leurs sels pharmaceutiquement acceptables.

**13.** Médicaments, caractérisés en ce qu'ils sont constitués par les nouvelles imidazobenzodiazépines telles que définies à l'une quelconque des revendications 2, 3, ou 4, ainsi que par leurs sels pharmaceutiquement acceptables.

**14.** Compositions pharmaceutiques, caractérisées en ce qu'elles renferment à titre de principe actif, l'un au moins des médicaments tels que définis à l'une quelconque des revendications 12 ou 13.

**15.** A titre de produits industriels nouveaux :

- Les produits de formule (VII) :

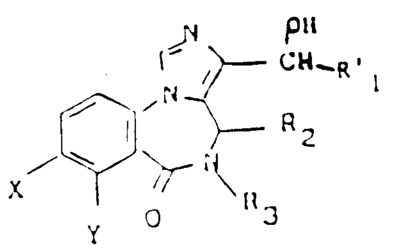

(VII)

dans laquelle $R'_1$, $R_2$, $R_3$, X et Y ont la signification déjà indiquée,

- Les produits de formule (XI) :

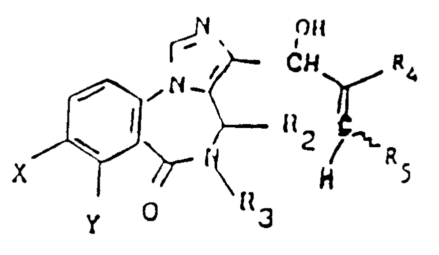

(XI)

dans laquelles $R_2$, $R_3$, $R_4$, $R_5$, X et Y ont la signification déjà indiquée,

- Les produits de formule (IX) :

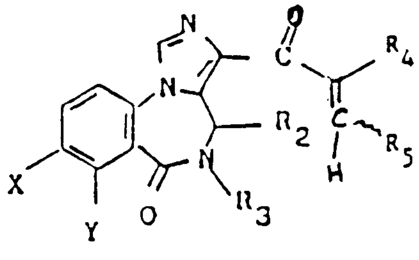

(IX)

dans laquelle $R_2$, $R_3$, $R_4$, $R_5$, ont la signification déjà indiquée,

- Les produits de formule (III) :

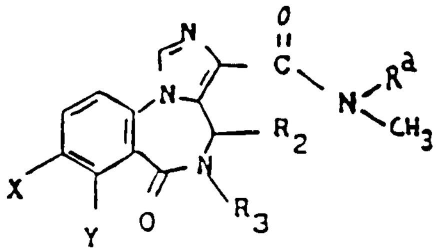

(III)

dans laquelle $R_2$, $R_3$, $R^a$, X et Y ont la signification déjà indiquée,

- Les produits de formule (XIII) :

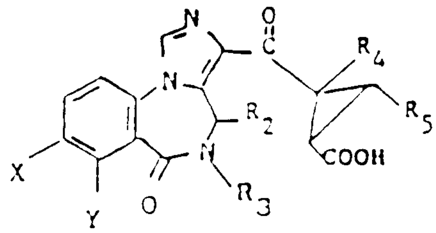

(XIII)

dans laquelle $R_2$, $R_3$, $R_4$, $R_5$, X et Y ont la signification déjà indiquée.

**Revendications pour l'Etat contractant suivant : ES**

**1.** Procédé de préparation des nouvelles imidazo benzodiazépines répondant à la formule générale (I) :

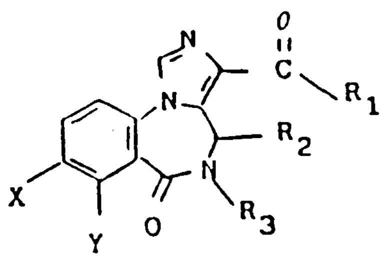

(I)

dans laquelle $R_1$ représente un radical phényle, cycloalcoyle renfermant de 4 à 6 atomes de carbone ou un groupement de formule (II) :

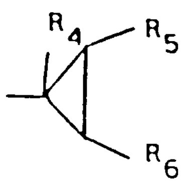

(II)

dans laquelle $R_4$ représente un atome d'hydrogène ou un radical alcoyle linéaire ou ramifié renfermant de 1 à 5 atomes de carbone, $R_5$ représente un atome d'hydrogène ou un radical alcoyle linéaire ou ramifié renfermant de 1 à 5 atomes de carbone, $R_6$ représente un atome d'hydrogène, un radical alcoyle linéaire ou ramifié renfermant de 1 à 5 atomes de carbone, un radical phényle, un atome d'halogène, un radical alkoxycarbonyle renfermant de 2 à 5 atomes de carbone, un radical cyano, un radical amido, un radical mono-ou dialcoylamido dont les groupements alcoyles linéaires ou ramifiés

renferment de 1 à 5 atomes de carbone, $R_2$ et $R_3$, identiques ou différents, représentent un atome d'hydrogène, un radical alcoyle linéaire ou ramifié, renfermant de 1 à 5 atomes de carbone, un radical cycloalcoyle renfermant de 3 à 5 atomes de carbone, ou $R_2$ et $R_3$ forment ensemble un groupement alkylène renfermant de 3 à 5 atomes de carbone, X et Y, identiques ou différents, représentent un atome d'hydrogène, un atome d'halogène, un radical alcoyle ou alcoxy renfermant de 1 à 3 atomes de carbone, un groupement nitro, azido, nitrile ou trifluorométhyle, ainsi que de leurs sels d'addition avec les acides minéraux ou organiques caractérisé en ce que :

a) pour préparer de produits répondant à la formule ($I_A$) :

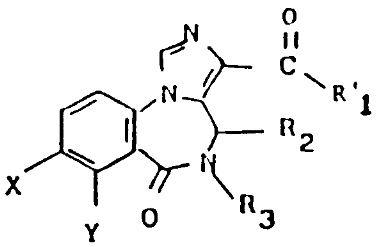

($I_A$)

dans laquelle, $R_2$, $R_3$, X et Y ont la signification déjà indiquée et $R'_1$ représente un radical phényle, cycloalcoyle renfermant de 4 à 6 atomes de carbone ou un groupement de formule II dans laquelle $R_4$ représente un atome d'hydrogène et $R_5$ et $R_6$ identiques ou différents représentent un atome d'hydrogène ou un radical alcoyle renfermant de 1 à 5 atomes de carbone, ou leurs sels,

- soit l'on fait réagir un produit de formule (V) :

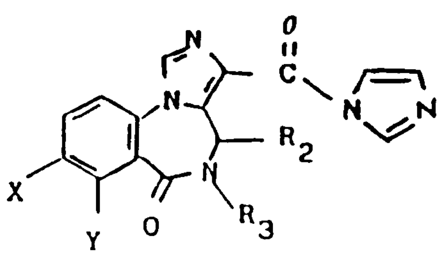

(V)

dans laquelle X, Y, $R_2$ et $R_3$ ont la signification indiquée précédemment, avec un produit de formule (VI) :

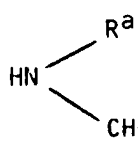

(VI)

dans laquelle $R^a$ représente un radical méthyle ou méthoxy ou un sel d'addition avec les acides, pour obtenir un produit de formule III :

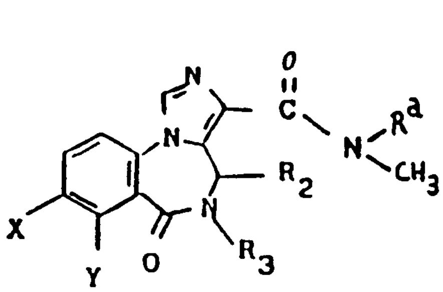

(III)

dans laquelle $R_2$, $R_3$, $R^a$, X et Y ont la signification déjà indiquée, puis fait réagir ledit produit

de formule III avec un produit de formule IV :

M - R'$_1$ (IV)

dans laquelle M représente un atome de métal alcalin tel qu'un atome de lithium ou un groupe -Mg-Hal dans lequel Hal représente un atome de chlore, de brome ou d'iode et R'$_1$ a la signification déjà indiquée pour obtenir le produit de formule $I_A$ attendu que l'on peut salifier le cas échéant,

- soit l'on fait réagir un produit de formule VIII :

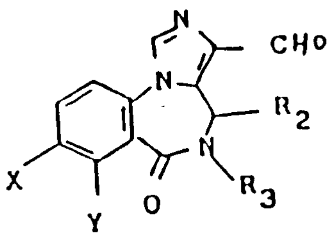

(VIII)

dans laquelle X, Y, $R_2$ et $R_3$ ont la signification déjà indiquée avec un produit de formule IV telle que définie ci-dessus pour obtenir un produit de formule (VII) :

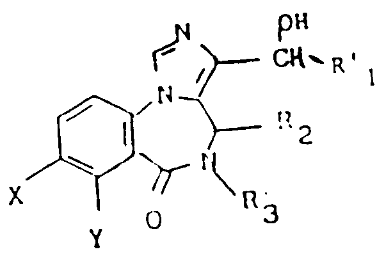

(VII)

dans laquelle R'$_1$, $R_2$, $R_3$, X et Y ont la signification déjà indiquée, puis oxyde le produit de formule (VII) ainsi obtenu, pour obtenir le produit de formule ($I_A$) attendu que l'on peut salifier le cas échéant,

b) Pour préparer des produits répondant à la formule ($I_B$) :

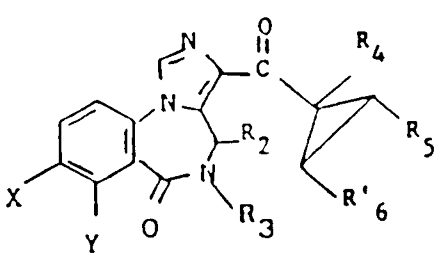

($I_B$)

dans laquelle $R_2$, $R_3$, $R_4$, $R_5$, X et Y ont la signification déjà indiquée et R'$_6$ représente un atome d'hydrogène, un atome d'halogène, un radical alcoyle renfermant de 1 à 5 atomes de carbone, un radical alkoxy carbonyle renfermant de 2 à 5 atomes de carbone, un radical phényle, cyano ou un groupement

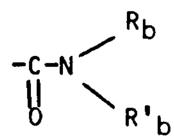

dans lequel $R_b$ et $R'_b$ identiques ou différents représentent un atome d'hydrogène ou un radical alcoyle renfermant de 1 à 5 atomes de carbone

- soit l'on fait réagir un produit de formule (III) telle que définie ci-dessus avec un composé de formule (X)

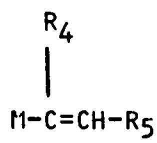

(X)

dans laquelle M, $R_4$ et $R_5$ ont la signification déjà indiquée pour obtenir un produit de formule (IX) :

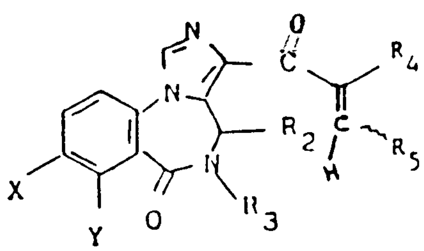

(IX)

dans laquelle $R_2$, $R_3$, $R_4$, $R_5$, X et Y ont la signification déjà indiquée,

- soit l'on fait réagir un produit de formule VIII telle que définie ci-dessus avec un composé de formule X pour obtenir un produit de formule (XI) :

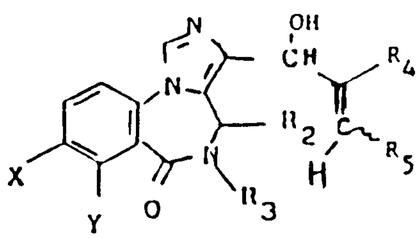

(XI)

dans laquelle $R_2$, $R_3$, $R_4$, $R_5$, X et Y ont la signification déjà indiquée, puis soumet le produit de formule (XI) ainsi obtenu, à l'action d'un agent d'oxydation pour obtenir un produit de formule (IX), produit de formule (IX) que l'on fait réagir avec un agent de cyclisation approprié pour obtenir un produit de formule ($I_B$) telle que définie ci-dessus, dans laquelle $R'_6$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, puis saponifie le cas échéant le produit obtenu dans lequel $R'_6$ représente un radical alcoxycarbonyle pour obtenir un produit de formule (XIII) :

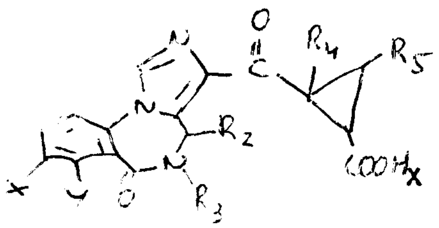

(XIII)

dans laquelle $R_2$, $R_3$, $R_4$, $R_5$, X et Y ont la signification déjà indiquée, puis fait réagir ledit produit de formule (XIII) avec une amine de formule (XII) :

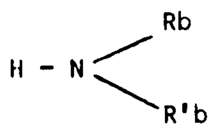

(XII)

dans laquelle Rb et R'b ont la signification déjà indiquée pour obtenir le produit correspondant de formule $I_B$ dans laquelle $R'_6$ représente un groupement de formule

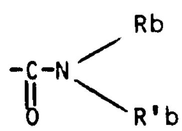

puis le cas échéant deshydrate ce dernier dans lequel Rb et R'b représentent un atome d'hydrogène pour obtenir le produit correspondant de formule $I_B$ dans laquelle $R'_6$ représente un radical cyano, et le cas échéant salifie les produits de formule $I_B$ ainsi obtenus.

**2.** Procédé selon la revendication 1 caractérisé en ce que l'on utilise un produit de formule IV dans laquelle $R'_1$ représente un radical cyclopropyle ou bien un produit de formule X dans laquelle $R_4$ et $R_5$ représentent un atome d'hydrogène.

**3.** Procédé selon la revendication 1 ou 2 caractérisé en ce que l'on utilise un produit de formule V ou VIII dans laquelle $R_2$ et $R_3$, identiques ou différents, représentent un atome d'hydrogène, un radical méthyle, X et Y, identiques ou différents, représentent un atome d'hydrogène, un atome de fluor ou de brome.

**4.** Procédé selon la revendication 1 ou 2 caractérisé en ce que l'on utilise un produit de formule V ou VIII dans laquelle $R_3$ représente un radical méthyle $R_2$ représente un atome d'hydrogène, X et Y représentent un atome d'hydrogène et un produit de formule IV dans laquelle $R'_1$ représente un radical cyclopropyle ou un produit de formule X dans laquelle $R_4$ et $R_5$ représentent un atome d'hydrogène.

**Revendications pour l'Etat contractant suivant : GR**

1. Procédé de préparation des nouvelles imidazo benzodiazépines répondant à la formule générale (I) :

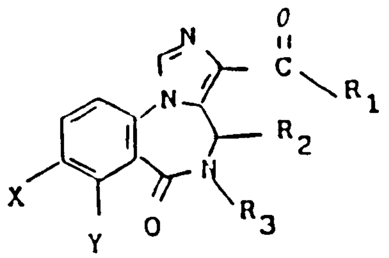

(I)

dans laquelle $R_1$ représente un radical phényle, cycloalcoyle renfermant de 4 à 6 atomes de carbone ou un groupement de formule (II) :

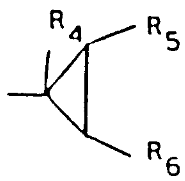

(II)

dans laquelle $R_4$ représente un atome d'hydrogène ou un radical alcoyle linéaire ou ramifié renfermant de 1 à 5 atomes de carbone, $R_5$ représente un atome d'hydrogène ou un radical alcoyle linéaire ou ramifié renfermant de 1 à 5 atomes de carbone, $R_6$ représente un atome d'hydrogène, un radical alcoyle linéaire ou ramifié renfermant de 1 à 5 atomes de carbone, un radical phényle, un atome d'halogène, un radical alkoxycarbonyle renfermant de 2 à 5 atomes de carbone, un radical cyano, un radical amido, un radical mono- ou dialcoylamido dont les groupements alcoyles linéaires ou ramifiés renferment de 1 à 5 atomes de carbone, $R_2$ et $R_3$, identiques ou différents, représentent un atome d'hydrogène, un radical alcoyle linéaire ou ramifié, renfermant de 1 à 5 atomes de carbone, un radical cycloalcoyle renfermant de 3 à 5 atomes de carbone, ou $R_2$ et $R_3$ forment ensemble un groupement alkylène renfermant de 3 à 5 atomes de carbone, X et Y, identiques ou différents, représentent un atome d'hydrogène, un atome d'halogène, un radical alcoyle ou alcoxy renfermant de 1 à 3 atomes de carbone, un groupement nitro, azido, nitrile ou trifluorométhyle, ainsi que de leurs sels d'addition avec les acides minéraux ou organiques caractérisé en ce que :

a) pour préparer des produits répondant à la formule ($I_A$) :

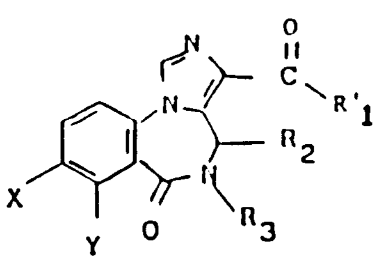

($I_A$)

dans laquelle, $R_2$, $R_3$, X et Y ont la signification déjà indiquée et $R'_1$ représente un radical phényle, cycloalcoyle renfermant de 4 à 6 atomes de carbone ou un groupement de formule II dans laquelle $R_4$ représente un atome d'hydrogène et $R_5$ et $R_6$ identiques ou différents représentent un atome d'hydrogène ou un radical alcoyle renfermant de 1 à 5 atomes de carbone, ou leurs sels :

- soit l'on fait réagir un produit de formule (V) :

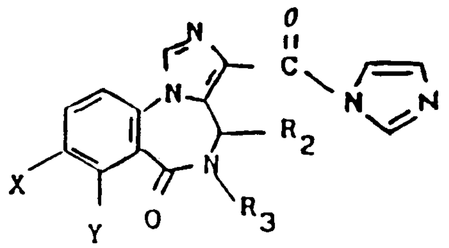

(V)

dans laquelle X, Y, $R_2$ et $R_3$ ont la signification indiquée précédemment, avec un produit de formule (VI) :

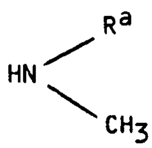

(VI)

dans laquelle $R^a$ représente un radical méthyle ou méthoxy ou un sel d'addition avec les acides, pour obtenir un produit de formule III :

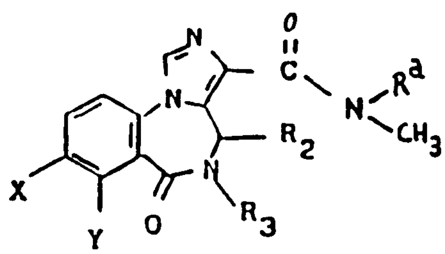

(III)

dans laquelle $R_2$, $R_3$, $R^a$, X et Y ont la signification déjà indiquée, puis fait réagir ledit produit de formule III avec un produit de formule IV :

M - $R'_1$ (IV)

dans laquelle M représente un atome de métal alcalin tel qu'un atome de lithium ou un groupe -Mg-Hal dans lequel Hal représente un atome de chlore, de brome ou d'iode et $R'_1$ a la signification déjà indiquée pour obtenir le produit de formule $I_A$ attendu que l'on peut salifier le cas échéant,

- soit l'on fait réagir un produit de formule VIII :

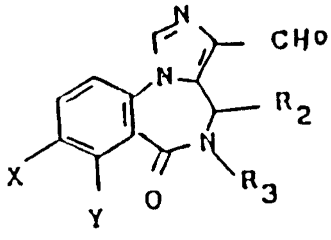

(VIII)

dans laquelle X, Y, $R_2$ et $R_3$ ont la signification déjà indiquée avec un produit de formule IV telle que définie ci-dessus pour obtenir un produit de formule (VII) :

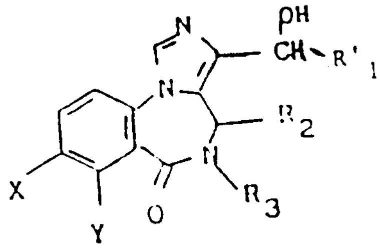

(VII)

dans laquelle $R'_1$, $R_2$, $R_3$, X et Y ont la signification déjà indiquée, puis oxyde le produit de formule (VII) ainsi obtenu, pour obtenir le produit de formule ($I_A$) attendu que l'on peut salifier le cas échéant,

b) Pour préparer des produits répondant à la formule ($I_B$) :

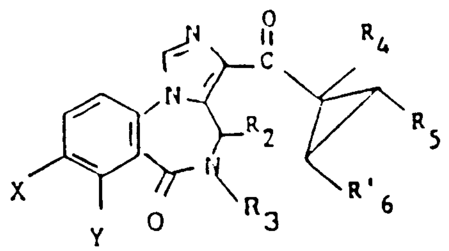

($I_B$)

dans laquelle $R_2$, $R_3$, $R_4$, $R_5$, X et Y ont la signification déjà indiquée et $R'_6$ représente un atome d'hydrogène, un atome d'halogène, un radical alcoyle renfermant de 1 à 5 atomes de carbone, un radical alkoxy carbonyle renfermant de 2 à 5 atomes de carbone, un radical phényle, cyano ou un groupement

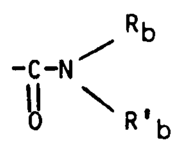

dans lequel $R_b$ et $R'_b$ identiques ou différents représentent un atome d'hydrogène ou un radical alcoyle renfermant de 1 à 5 atomes de carbone :

- soit l'on fait réagir un produit de formule (III) telle que définie ci-dessus avec un composé de formule (X)

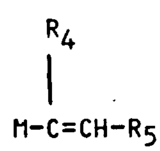

(X)

dans laquelle M, $R_4$ et $R_5$ ont la signification déjà indiquée pour obtenir un produit de formule (IX) :

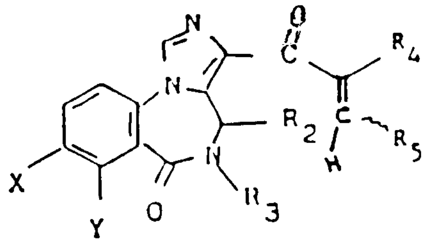

(IX)

dans laquelle $R_2$, $R_3$, $R_4$, $R_5$, X et Y ont la signification déjà indiquée,

- soit l'on fait réagir un produit de formule VIII telle que définie ci-dessus avec un composé de formule X pour obtenir un produit de formule (XI) :

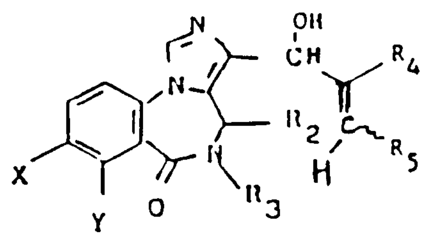

(XI)

dans laquelle $R_2$, $R_3$, $R_4$, $R_5$, X et Y ont la signification déjà indiquée, puis soumet le produit de formule (XI) ainsi obtenu, à l'action d'un agent d'oxydation pour obtenir un produit de formule (IX), produit de formule (IX) que l'on fait réagir avec un agent de cyclisation approprié pour obtenir un produit de formule ($I_B$) telle que définie ci-dessus, dans laquelle $R'_6$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, puis saponifie le cas échéant le produit obtenu dans lequel $R'_6$ représente un radical alcoxycarbonyle pour obtenir un produit de formule (XIII) :

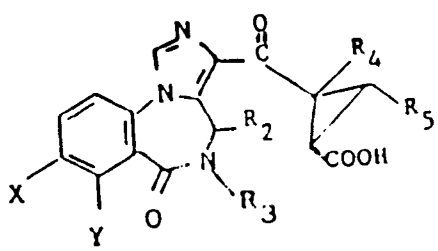

(XIII)

dans laquelle $R_2$, $R_3$, $R_4$, $R_5$, X et Y ont la signification déjà indiquée, puis fait réagir ledit produit de formule (XIII) avec une amine de formule (XII) :

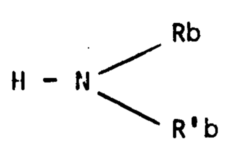

(XII)

dans laquelle Rb et R'b ont la signification déjà indiquée pour obtenir le produit correspondant de formule $I_B$ dans laquelle $R'_6$ représente un groupement de formule

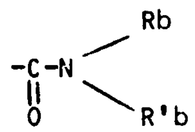

puis le cas échéant deshydrate ce dernier dans lequel Rb et R'b représentent un atome d'hydrogène pour obtenir le produit correspondant de formule $I_B$ dans laquelle $R'_6$ représente un radical cyano, et le cas échéant salifie les produits de formule $I_B$ ainsi obtenus.

**2.** Procédé selon la revendication 1 caractérisé en ce que l'on utilise un produit de formule IV dans laquelle $R'_1$ représente un radical cyclopropyle ou bien un produit de formule X dans laquelle $R_4$ et $R_5$ représentent un atome d'hydrogène.

**3.** Procédé selon la revendication 1 ou 2 caractérisé en ce que l'on utilise un produit de formule V ou VIII dans laquelle $R_2$ et $R_3$, identiques ou différents, représentent un atome d'hydrogène, un radical méthyle, X et Y, identiques ou différents, représentent un atome d'hydrogène, un atome de fluor ou de brome.

**4.** Procédé selon la revendication 1 ou 2 caractérisé en ce que l'on utilise un produit de formule V ou VIII dans laquelle $R_3$ représente un radical méthyle $R_2$ représente un atome d'hydrogène, X et Y représentent un atome d'hydrogène et un produit de formule IV dans laquelle $R'_1$ représente un radical cyclopropyle ou un produit de formule X dans laquelle $R_4$ et $R_5$ représentent un atome d'hydrogène.

**5.** A titre de produits industriels nouveaux les produits de formule VII, XI, IX, III et XIII tels que définis à la revendication 1.

**6.** Procédé de préparation de compositions pharmaceutiques caractérisé en ce que l'on met à titre de principe actif l'un au moins des composés de formule I tels que définis à la revendication 1 ou l'un au moins de leurs sels d'addition avec les acides minéraux ou organiques pharmaceutiquement acceptables sous une forme destinée à cet usage.

**Claims**

**Claims for the following Contracting States : AT, BE, CH, DE, FR, IT, LI, LU, NL, SE**

**1.** New imidazo benzodiazepines corresponding to general formula (I):

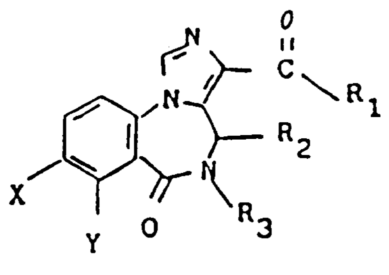

(I)

in which $R_1$ represents a phenyl or cycloalkyl radical containing 4 to 6 carbon atoms or a group of formula (II):

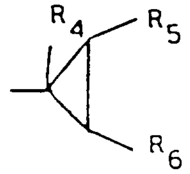

(II)

in which $R_4$ represents a hydrogen atom or a linear or branched alkyl radical containing 1 to 5 carbon atoms, $R_5$ represents a hydrogen atom or a linear or branched alkyl radical containing 1 to 5 carbon atoms, $R_6$ represents a hydrogen atom, a linear or branched alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the linear or branched alkyl groups of which contain 1 to 5 carbon atoms, $R_2$ and $R_3$, identical or different, represent a hydrogen atom, a linear or branched alkyl radical containing 1 to 5 carbon atoms, a cycloalkyl radical containing 3 to 5 carbon atoms, or $R_2$ and $R_3$ form together an alkylene group containing 3 to 5 carbon atoms, X and Y identical or different, represent a hydrogen atom, a halogen atom, an alkyl or alkoxy radical containing 1 to 3 carbon atoms, a nitro, azido, nitrile or trifluoromethyl group, as well as their addition salts with mineral or organic acids.

**2.** New imidazo benzodiazepines as defined by formula (I) of claim 1, characterized in that in the said formula (I), $R_1$ represents a cyclopropyl group and $R_2$, $R_3$, X and Y have the meaning already indicated, as well as their addition salts with mineral or organic acids.

**3.** New imidazo benzodiazepines according to claim 1 or 2, characterized in that in the said formula (I), $R_1$ represents a cyclopropyl group, $R_2$ and $R_3$, identical or different, represent a hydrogen atom, a methyl radical, X and Y, identical or different, represent a hydrogen atom, a fluorine or bromine atom, as well as their addition salts with mineral or organic acids.

**4.** 3-(5,6-dihydro-5-methyl-6-oxo-4H-imidazo [1,5a] [1,4] benzodiazepinyl)-cyclopropylmethanone.

**5.** Preparation process for new imidazo benzodiazepines according to claim 1 corresponding to formula ($I_A$):

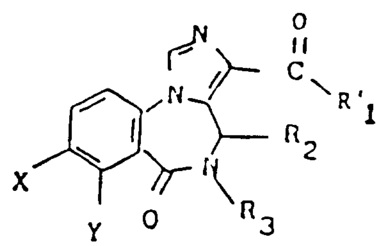

($I_A$)

in which $R_2$, $R_3$, X and Y have the meaning already indicated and $R'_1$ represent a phenyl or cycloalkyl radical containing 4 to 6 carbon atoms or a group of formula II in which $R_4$ represents a hydrogen atom and $R_5$ and $R_6$, identical or different, represent a hydrogen atom or an alkyl radical containing 1 to 5 carbon atoms, as well as their salts, characterized in that:

- either a product of formula (V):

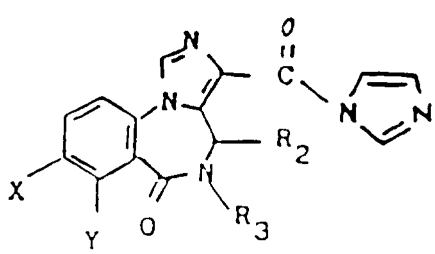

(V)

in which X, Y, $R_2$ and $R_3$ have the meaning indicated previously, is reacted with a product of formula (VI):

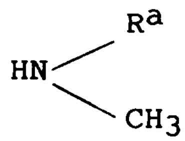

(VI)

in which $R^a$ represents a methyl or methoxy radical or an addition salt with acids, in order to obtain a product of formula (III):

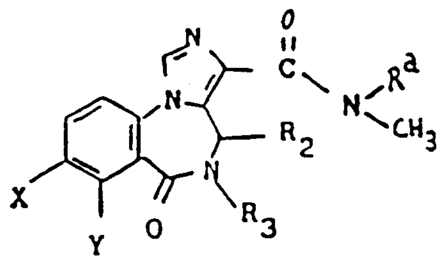

(III)

in which $R_2$, $R_3$, $R^a$, X and Y have the meaning already indicated, then the said product of formula (III) is reacted with a product of formula (IV):

M - $R'_1$ (IV)

in which M represents an alkali metal atom such as a lithium atom or a -Mg-Hal group in which Hal represents a chlorine, bromine or iodine atom and $R'_1$ has the meaning already indicated, in order to obtain the expected product of formula ($I_A$) which can be salified if appropriate,

- or a product of formula (VIII):

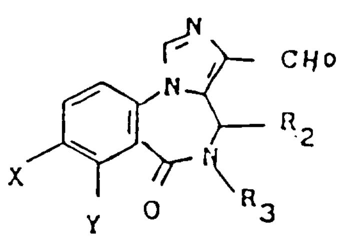

(VIII)

in which X, Y, $R_2$ and $R_3$ have the meaning already indicated, is reacted with a product of formula (IV) as defined above, in order to obtain a product of formula (VII):

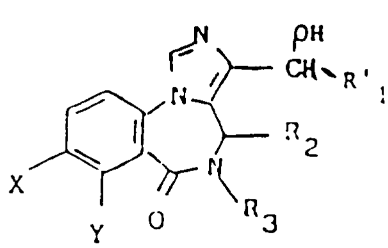

(VII)

in which $R'_1$, $R_2$, $R_3$, X and Y have the meaning already indicated, then the product of formula (VII) thus obtained is oxidized in order to obtain the expected product of formula ($I_A$) which can be salified if appropriate.

**6.** Preparation process according to claim 5, characterized in that:

- the reaction of the product of formula (V) with the product of formula (VI) is advantageously carried out in dimethylformamide;
- the reaction of the product of formula (III) or (VIII) with the product of formula (IV) is carried out in anhydrous conditions, in an organic solvent such as tetrahydrofuran;
- the oxidation of the product of formula (VII) is preferably carried out with manganese dioxide, nitric acid, ferric chloride or chromium oxide in the presence of pyridine or also by the Oppenauer method or finally by dehydrogenation in the presence of a catalyst based on copper.

**7.** Preparation process for new imidazo benzodiazepines as defined in formula (I) of claim 1 corresponding to formula ($I_B$):

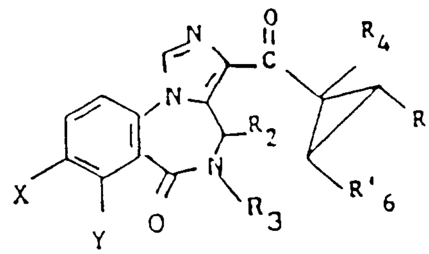

($I_B$)

in which $R_2$, $R_3$, $R_4$, $R_5$, X and Y have the meaning already indicated and $R'_6$ represents a hydrogen atom, a halogen atom, an alkyl radical containing 1 to 5 carbon atoms, an alkoxy carbonyl radical containing 2 to 5 carbon atoms, a phenyl or cyano radical or a

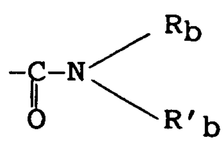

group in which $R_b$ and $R'_b$, identical or different, represent a hydrogen atom or an alkyl radical containing 1 to 5 carbon atoms, characterized in that:

- either a product of formula (III) as defined above is reacted with a compound of formula (X):

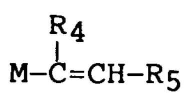

(X)

in which M, $R_4$ and $R_5$ have the meaning already indicated, in order to obtain a product of formula (IX):

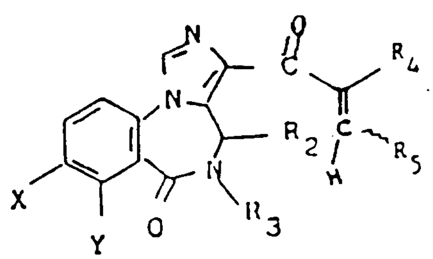

(IX)

in which $R_2$, $R_3$, $R_4$, $R_5$, X and Y have the meaning already indicated,

- or a product of formula (VIII) as defined above is reacted with a compound of formula (X) in order to obtain a product of formula (XI):

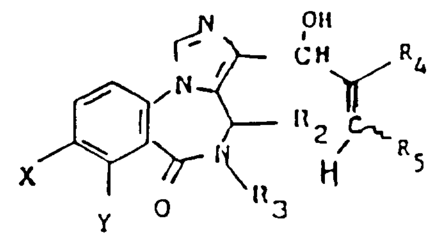

(XI)

in which $R_2$, $R_3$, $R_4$, $R_5$, X and Y have the meaning already indicated, then the product of formula (XI) thus obtained is subjected to the action of an oxidation agent in order to obtain a product of formula (IX), which product of formula (IX) is reacted with an appropriate cyclization agent in order to obtain a product of formula ($I_B$) as defined above, in which $R'_6$ represents a hydrogen atom, a halogen atom, an alkyl radical containing 1 to 5 carbon atoms, an alkoxy carbonyl radical containing 2 to 5 carbon atoms or a phenyl radical, then if appropriate the product obtained in which $R'_6$ represents an alkoxycarbonyl radical is saponified in order to obtain a product of formula (XIII):

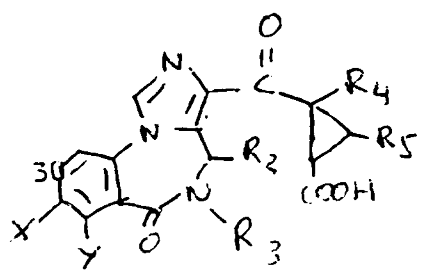

(XIII)

in which $R_2$, $R_3$, $R_4$, $R_5$, X and Y have the meaning already indicated, then the said product of formula (XIII) is reacted with an amine of formula (XII):

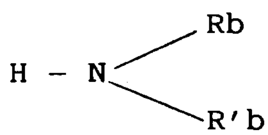

(XII)

in which Rb and R'b have the meaning already indicated, in order to obtain the corresponding product of formula ($I_B$) in which $R'_6$ represents a group of formula

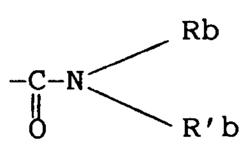

,

then if appropriate this product in which Rb and R'b represent a hydrogen atom is dehydrated in order to obtain the corresponding product of formula ($I_B$) in which $R'_6$ represents a cyano radical, and if appropriate the products of formula ($I_B$) thus obtained are salified.

**8.** Preparation process according to claim 7, characterized in that the reaction of the products of formulae (III) and (VIII) with the product of formula (X) is carried out in anhydrous conditions, in an organic solvent such as tetrahydrofuran.

**9.** Preparation process according to claim 7, characterized in that the oxidation of the product of formula (XI) is preferably carried out with manganese dioxide, nitric acid, ferric chloride or chromium oxide, in the presence of pyridine, or also by the Oppenauer method or finally by dehydrogenation in the

presence of a catalyst based on copper.

**10.** Preparation process according to claim 7, characterized in that:

- the appropriate cyclization agent is a reagent capable of introducing the

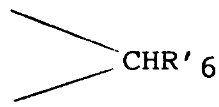

  group at the level of the double bond;
- when it is desired to obtain a product of formula ($I_B$) in which $R_4$ represents an alkyl radical containing 1 to 5 carbon atoms, $R_5$ represents a hydrogen atom and $R'_6$ represents a hydrogen atom or an alkyl radical containing 1 to 5 carbon atoms, the cyclization is advantageously carried out using a trialkyloxosulphonium iodide operating in an organic solvent such as dimethylformamide;
- when it is desired to obtain a product of formula ($I_B$) in which $R_4$ represents a hydrogen atom and $R_5$ represents an alkyl radical containing 1 to 5 carbon atoms and $R'_6$ represents a hydrogen atom or an alkyl radical containing 1 to 5 carbon atoms, the cyclization is advantageously carried out using a dimethylamino alkyl phenyl oxosulphonium tetrafluoroborate operating in an organic solvent such as dimethyl formamide;
- when it is desired to obtain a product of formula ($I_B$) in which $R_4$ represents a hydrogen atom and $R'_6$ represents an alkoxy carbonyl or phenyl group, the cyclization is advantageously carried out using a dimethylsulphuranylidene acetate or a benzyldimethylsulphonium anion operating in an organic solvent such as chloroform;
- when it is desired to obtain a product of formula ($I_B$) in which $R_4$ represents a hydrogen atom and $R'_6$ represents a halogen atom, the cyclization is advantageously carried out using a dimethyl amino phenyl oxosulphonium halogeno methylylide operating in an organic solvent such as dimethyl formamide.

**11.** Preparation process according to claim 7, characterized in that:

- the saponification of the product of formula ($I_B$) in which $R'_6$ represents an alkoxycarbonyl radical is preferably carried out using an alkaline hydroxide such as sodium hydroxide;
- the reaction of the product of formula ($I_B$) with the amine of formula (XII) is preferably carried out in an anhydrous organic solvent in the presence of carbonyldiimidazole;
- the dehydration of the product of formula ($I_B$) is preferably carried out using a strong acid anhydride such as trifluoroacetic acid anhydride in an organic solvent such as dichloromethane.

**12.** Medicaments, characterized in that they are constituted by new imidazo benzodiazepines as defined by formula (I) of claim 1 as well as by their pharmaceutically acceptable salts.

**13.** Medicaments, characterized in that they are constituted by new imidazo benzodiazepines as defined in any one of claims 2, 3 or 4, as well as by their pharmaceutically acceptable salts.

**14.** Pharmaceutical compositions, characterized in that they contain as active ingredient at least one of the medicaments as defined in any one of claims 12 or 13.

**15.** As new industrial products:

- the products of formula (VII):

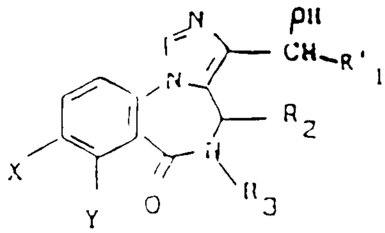

(VII)

in which $R'_1$, $R_2$, $R_3$, X and Y have the meaning already indicated,

- the products of formula (XI):

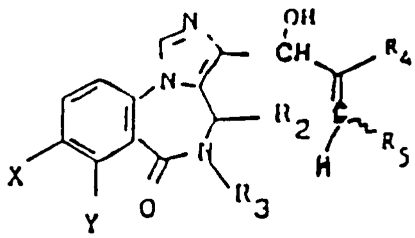

(XI)

in which $R_2$, $R_3$, $R_4$, $R_5$, X and Y have the meaning already indicated,

- the products of formula (IX):

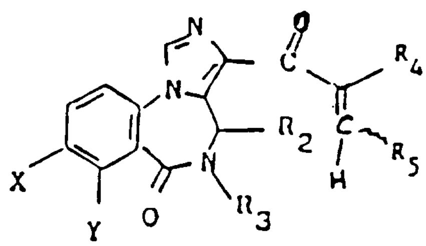

(IX)

in which $R_2$, $R_3$, $R_4$, $R_5$ have the meaning already indicated,

- the products of formula (III):

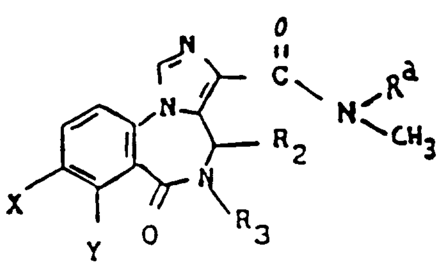

(III)

in which $R_2$, $R_3$, $R^a$, X and Y have the meaning already indicated,

- the products of formula (XIII):

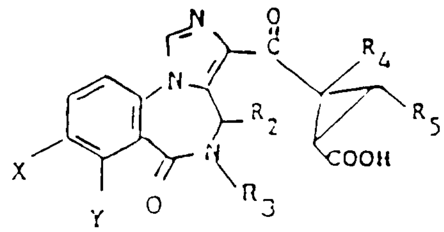

(XIII)

in which $R_2$, $R_3$, $R_4$, $R_5$, X and Y have the meaning already indicated.

**Claims for the following Contracting State : ES**

**1.** Preparation process for new imidazo benzodiazepines corresponding to general formula (I):

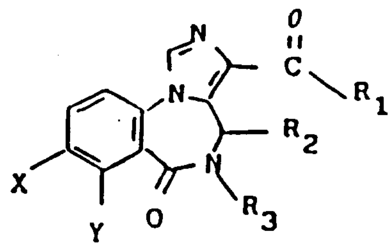

(I)

in which $R_1$ represents a phenyl or cycloalkyl radical containing 4 to 6 carbon atoms or a group of formula (II):

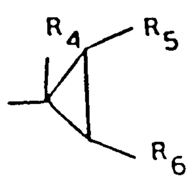

(II)

in which $R_4$ represents a hydrogen atom or a linear or branched alkyl radical containing 1 to 5 carbon atoms, $R_5$ represents a hydrogen atom or a linear or branched alkyl radical containing 1 to 5 carbon atoms, $R_6$ represents a hydrogen atom, a linear or branched alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the linear or branched alkyl groups of which contain 1 to 5 carbon atoms, $R_2$ and $R_3$, identical or different, represent a hydrogen atom, a linear or branched alkyl radical containing 1 to 5 carbon atoms, a cycloalkyl radical containing 3 to 5 carbon atoms, or $R_2$ and $R_3$ form together an alkylene group containing 3 to 5 carbon atoms, X and Y identical or different, represent a hydrogen atom, a halogen atom, an alkyl or alkoxy radical containing 1 to 3 carbon atoms, a nitro, azido, nitrile or trifluoromethyl group, as well as their addition salts with mineral or organic acids, characterized in that:

a) in order to prepare products corresponding to formula ($I_A$):

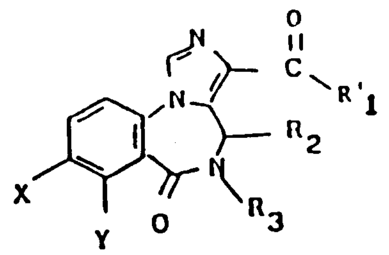

($I_A$)

in which $R_2$, $R_3$, X and Y have the meaning already indicated and $R'_1$ represent a phenyl or cycloalkyl radical containing 4 to 6 carbon atoms or a group of formula II in which $R_4$ represents a hydrogen atom and $R_5$ and $R_6$, identical or different, represent a hydrogen atom or an alkyl radical containing 1 to 5 carbon atoms, or their salts

- either a product of formula (V):

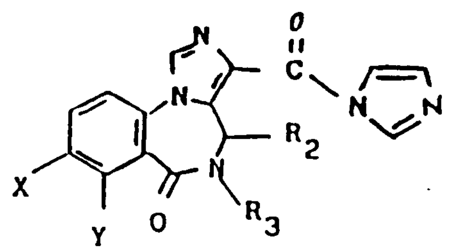

(V)

in which X, Y, $R_2$ and $R_3$ have the meaning indicated previously, is reacted with a product of formula (VI):

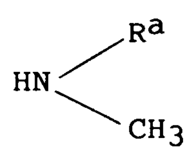

(VI)

in which $R^a$ represents a methyl or methoxy radical or an addition salt with acids, in order to obtain a product of formula (III):

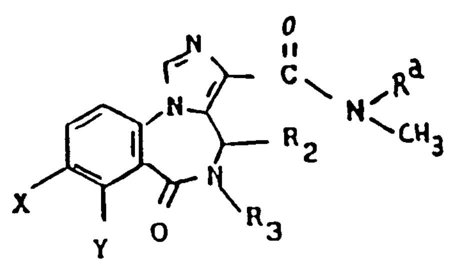

(III)

in which $R_2$, $R_3$, $R^a$, X and Y have the meaning already indicated, then the said product of formula (III) is reacted with a product of formula (IV):

M - $R'_1$ (IV)

in which M represents an alkali metal atom such as a lithium atom or a -Mg-Hal group in which Hal represents a chlorine, bromine or iodine atom and $R'_1$ has the meaning already indicated,

in order to obtain the expected product of formula ($I_A$) which can be salified if appropriate,

- or a product of formula (VIII):

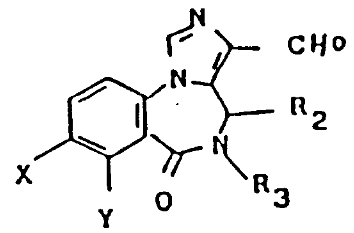

(VIII)

in which X, Y, $R_2$ and $R_3$ have the meaning already indicated, is reacted with a product of formula (IV) as defined above, in order to obtain a product of formula (VII):

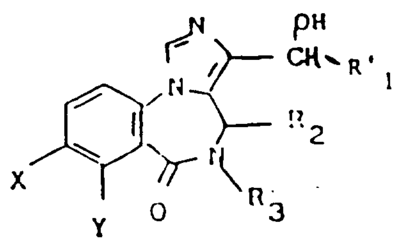

(VII)

in which $R'_1$, $R_2$, $R_3$, X and Y have the meaning already indicated, then the product of formula (VII) thus obtained is oxidized in order to obtain the expected product of formula ($I_A$) which can be salified if appropriate,

b) in order to prepare products corresponding to formula ($I_B$):

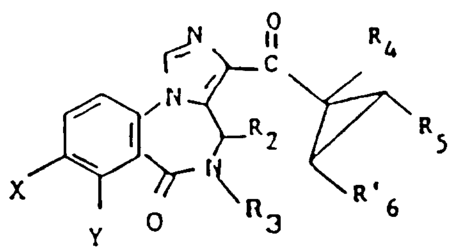

($I_B$)

in which $R_2$, $R_3$, $R_4$, $R_5$, X and Y have the meaning already indicated and $R'_6$ represents a hydrogen atom, a halogen atom, an alkyl radical containing 1 to 5 carbon atoms, an alkoxy carbonyl radical containing 2 to 5 carbon atoms, a phenyl or cyano radical or a

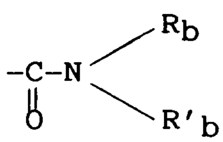

group in which $R_b$ and $R'_b$, identical or different, represent a hydrogen atom or an alkyl radical containing 1 to 5 carbon atoms

- either a product of formula (III) as defined above is reacted with a compound of formula (X):

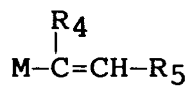

(X)

in which M, $R_4$ and $R_5$ have the meaning already indicated, in order to obtain a product of formula (IX):

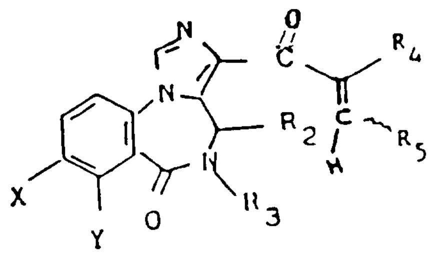

(IX)

in which $R_2$, $R_3$, $R_4$, $R_5$, X and Y have the meaning already indicated,

- or a product of formula (VIII) as defined above is reacted with a compound of formula (X) in order to obtain a product of formula (XI):

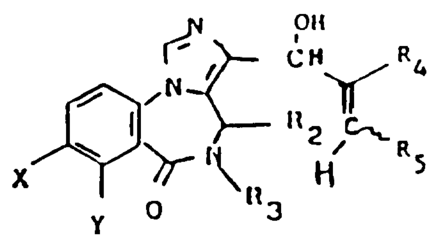

(XI)

in which $R_2$, $R_3$, $R_4$, $R_5$, X and Y have the meaning already indicated, then the product of formula (XI) thus obtained is subjected to the action of an oxidation agent in order to obtain a product of formula (IX), which product of formula (IX) is reacted with an appropriate cyclization agent in order to obtain a product of formula ($I_B$) as defined above, in which $R'_6$ represents a hydrogen atom, a halogen atom, an alkyl radical containing 1 to 5 carbon atoms, an alkoxy carbonyl radical containing 2 to 5 carbon atoms or a phenyl radical, then if appropriate the product obtained in which $R'_6$ represents an alkoxycarbonyl radical is saponified in order to obtain a product of formula (XIII):

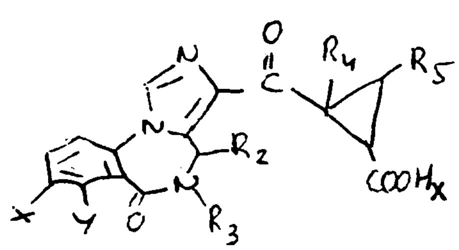

(XIII)

in which $R_2$, $R_3$, $R_4$, $R_5$, X and Y have the meaning already indicated, then the said product of formula (XIII) is reacted with an amine of formula (XII):

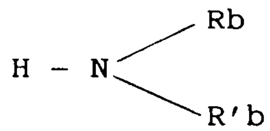

(XII)

in which Rb and R'b have the meaning already indicated, in order to obtain the corresponding product of formula ($I_B$) in which $R'_6$ represents a group of formula

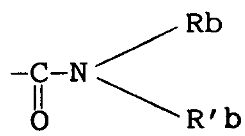

,

then if appropriate this product in which Rb and R'b represent a hydrogen atom is dehydrated in order to obtain the corresponding product of formula ($I_B$) in which $R'_6$ represents a cyano radical, and if appropriate the products of formula ($I_B$) thus obtained are salified.

2. Process according to claim 1, characterized in that a product of formula (IV) is used in which $R'_1$ represents a cyclopropyl radical or a product of formula (X) in which $R_4$ and $R_5$ represent a hydrogen atom.

3. Process according to claim 1 or 2, characterized in that a product of formula (V) or (VIII) is used in which $R_2$ and $R_3$, identical or different, represent a hydrogen atom, a methyl radical, X and Y, identical or different, represent a hydrogen atom, a fluorine or bromine atom.

4. Process according to claim 1 or 2, characterized in that a product of formula (V) or (VIII) is used in which $R_3$ represents a methyl radical, $R_2$ represents a hydrogen atom, X and Y represent a hydrogen atom and a product of formula (IV) in which $R'_1$ represents a cyclopropyl radical or a product of formula (X) in which $R_4$ and $R_5$ represent a hydrogen atom.

**Claims for the following Contracting State : GR**

1. Preparation process for new imidazo benzodiazepines corresponding to general formula (I):

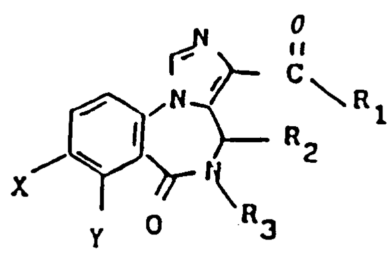

(I)

in which $R_1$ represents a phenyl or cycloalkyl radical containing 4 to 6 carbon atoms or a group of formula (II):

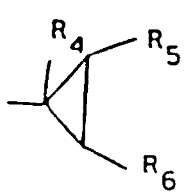

(II)

in which $R_4$ represents a hydrogen atom or a linear or branched alkyl radical containing 1 to 5 carbon atoms, $R_5$ represents a hydrogen atom or a linear or branched alkyl radical containing 1 to 5 carbon atoms, $R_6$ represents a hydrogen atom, a linear or branched alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the linear or branched alkyl groups of which contain 1 to 5 carbon atoms, $R_2$ and $R_3$, identical or different, represent a hydrogen atom, a linear or branched alkyl radical containing 1 to 5 carbon atoms, a cycloalkyl radical containing 3 to 5 carbon atoms, or $R_2$ and $R_3$ form together an alkylene group containing 3 to 5 carbon atoms, X and Y identical or different, represent a hydrogen atom, a halogen atom, an alkyl or alkoxy radical containing 1 to 3 carbon atoms, a nitro, azido, nitrile or trifluoromethyl group, as well as their addition salts with mineral or organic acids, characterized in that:

a) in order to prepare products corresponding to formula ($I_A$):

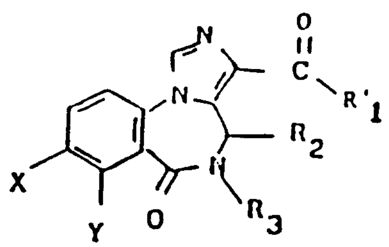

($I_A$)

in which $R_2$, $R_3$, X and Y have the meaning already indicated and $R'_1$ represent a phenyl or cycloalkyl radical containing 4 to 6 carbon atoms or a group of formula II in which $R_4$ represents a hydrogen atom and $R_5$ and $R_6$, identical or different, represent a hydrogen atom or an alkyl radical containing 1 to 5 carbon atoms, or their salts

- either a product of formula (V):

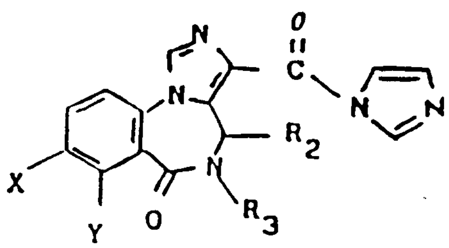

(V)

in which X, Y, $R_2$ and $R_3$ have the meaning indicated previously, is reacted with a product of formula (VI):

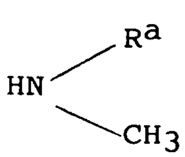

(VI)

in which $R^a$ represents a methyl or methoxy radical or an addition salt with acids, in order to obtain a product of formula (III):

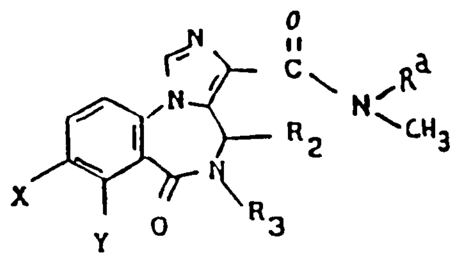

(III)

in which $R_2$, $R_3$, $R^a$, X and Y have the meaning already indicated, then the said product of formula (III) is reacted with a product of formula (IV):

M - R'$_1$ (IV)

in which M represents an alkali metal atom such as a lithium atom or a -Mg-Hal group in which Hal represents a chlorine, bromine or iodine atom and R'$_1$ has the meaning already indicated, in order to obtain the expected product of formula ($I_A$) which can be salified if appropriate,

- or a product of formula (VIII):

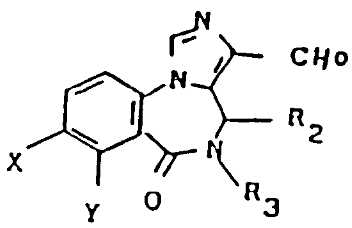

(VIII)

in which X, Y, $R_2$ and $R_3$ have the meaning already indicated, is reacted with a product of formula (IV) as defined above, in order to obtain a product of formula (VII):

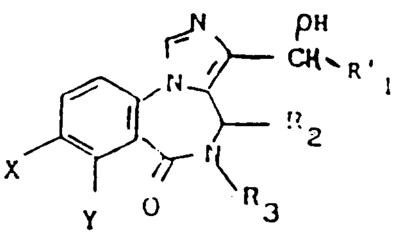

(VII)

in which R'$_1$, $R_2$, $R_3$, X and Y have the meaning already indicated, then the product of formula (VII) thus obtained is oxidized in order to obtain the expected product of formula ($I_A$) which can be salified if appropriate,

b) in order to prepare products corresponding to formula ($I_B$):

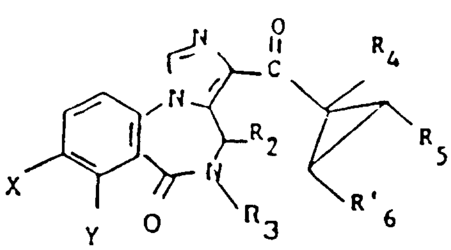

($I_B$)

in which $R_2$, $R_3$, $R_4$, $R_5$, X and Y have the meaning already indicated and R'$_6$ represents a hydrogen

atom, a halogen atom, an alkyl radical containing 1 to 5 carbon atoms, an alkoxy carbonyl radical containing 2 to 5 carbon atoms, a phenyl or cyano radical or a

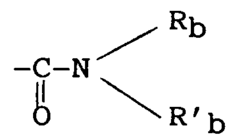

group in which $R_b$ and $R'_b$, identical or different, represent a hydrogen atom or an alkyl radical containing 1 to 5 carbon atoms

- either a product of formula (III) as defined above is reacted with a compound of formula (X):

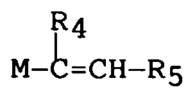

(X)

in which M, $R_4$ and $R_5$ have the meaning already indicated, in order to obtain a product of formula (IX):

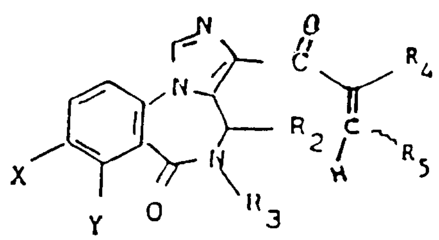

(IX)

in which $R_2$, $R_3$, $R_4$, $R_5$, X and Y have the meaning already indicated,

- or a product of formula (VIII) as defined above is reacted with a compound of formula (X) in order to obtain a product of formula (XI):

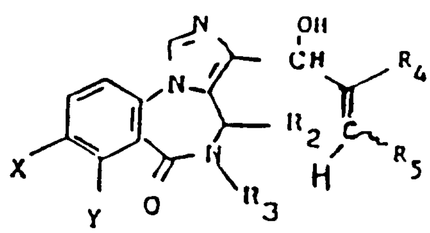

(XI)

in which $R_2$, $R_3$, $R_4$, $R_5$, X and Y have the meaning already indicated, then the product of formula (XI) thus obtained is subjected to the action of an oxidation agent in order to obtain a product of formula (IX), which product of formula (IX) is reacted with an appropriate cyclization agent in order to obtain a product of formula ($I_B$) as defined above, in which $R'_6$ represents a hydrogen atom, a halogen atom, an alkyl radical containing 1 to 5 carbon atoms, an alkoxy carbonyl radical containing 2 to 5 carbon atoms or a phenyl radical, then if appropriate the product obtained in which $R'_6$ represents an alkoxycarbonyl radical is saponified in order to obtain a product of formula (XIII):

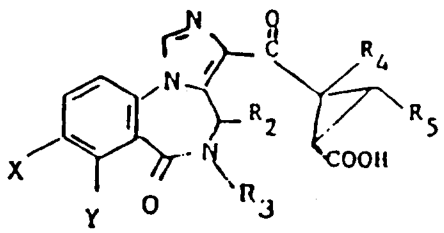

(XIII)

in which $R_2$, $R_3$, $R_4$, $R_5$, X and Y have the meaning already indicated, then the said product of formula (XIII) is reacted with an amine of formula (XII):

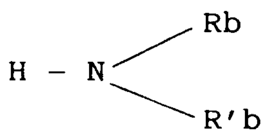

(XII)

in which Rb and R'b have the meaning already indicated, in order to obtain the corresponding product of formula ($I_B$) in which $R'_6$ represents a group of formula

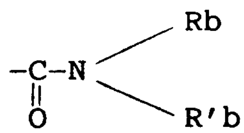

,

then if appropriate this product in which Rb and R'b represent a hydrogen atom is dehydrated in order to obtain the corresponding product of formula ($I_B$) in which $R'_6$ represents a cyano radical, and if appropriate the products of formula ($I_B$) thus obtained are salified.

**2.** Process according to claim 1, characterized in that a product of formula (IV) is used in which $R'_1$ represents a cyclopropyl radical or a product of formula (X) in which $R_4$ and $R_5$ represent a hydrogen atom.

**3.** Process according to claim 1 or 2, characterized in that a product of formula (V) or (VIII) is used in which $R_2$ and $R_3$, identical or different, represent a hydrogen atom, a methyl radical, X and Y, identical or different, represent a hydrogen atom, a fluorine or bromine atom.

**4.** Process according to claim 1 or 2, characterized in that a product of formula (V) or (VIII) is used in which $R_3$ represents a methyl radical, $R_2$ represents a hydrogen atom, X and Y represent a hydrogen atom and a product of formula (IV) in which $R'_1$ represents a cyclopropyl radical or a product of formula (X) in which $R_4$ and $R_5$ represent a hydrogen atom.

**5.** As new industrial products, the products of formulae (VII), (XI), (IX), (III) and (XIII) as defined in claim 1.

**6.** Preparation process for pharmaceutical compositions characterized in that at least one of the compounds of formula (I) as defined in claim 1 or at least one of their addition salts with pharmaceutically acceptable mineral or organic acids is used as active ingredient in a form intended for this use.

**Patentansprüche**

**Patentansprüche für folgende Vertragsstaaten : AT, BE, CH, DE, FR, IT, LI, LU, NL, SE**

1. Neue Imidazobenzodiazepine der allgemeinen Formel (I):

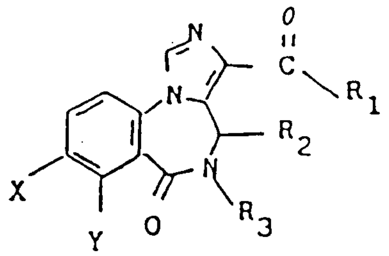

(I)

worin $R_1$ für einen Phenylrest, einen Cycloalkylrest mit 4 bis 6 Kohlenstoffatomen oder eine Gruppe der Formel (II);

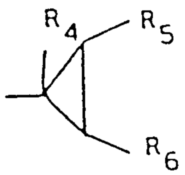

(II)

worin $R_4$ für ein Wasserstoffatom oder einen linearen oder verzweigten Alkylrest mit 1 bis 5 Kohlenstoffatomen steht, $R_5$ für ein Wasserstoffatom oder einen linearen oder verzweigten Alkylrest mit 1 bis 5 Kohlenstoffatomen steht, $R_6$ für ein Wasserstoffatom, einen linearen oder verzweigten Alkylrest mit 1 bis 5 Kohlenstoffatomen, einen Phenylrest, ein Halogenatom, einen Alkoxycarbonylrest mit 2 bis 5 Kohlenstoffatomen, eine Cyanogruppe, eine Amidogruppe, eine Mono- oder Dialkylamidogruppe, deren lineare oder verzweigte Alkylgruppen 1 bis 5 Kohlenstoffatome umfassen, steht, steht, $R_2$ und $R_3$, die gleich oder verschieden sind, für ein Wasserstoffatom, einen linearen oder verzweigten Alkylrest mit 1 bis 5 Kohlenstoffatomen, einen Cycloalkylrest mit 3 bis 5 Kohlenstoffatomen stehen, oder $R_2$ und $R_3$ zusammen einen Alkylenrest mit 3 bis 5 Kohlenstoffatomen bilden, X und Y, die gleich oder verschieden sind, für ein Wasserstoffatom, ein Halogenatom, einen Alkyl- oder Alkoxyrest mit 1 bis 3 Kohlenstoffatomen, eine Nitro-, Azido-, Nitril- oder Trifluormethylgruppe stehen, sowie ihre Additionssalze mit mineralischen oder organischen Säuren.

2. Neue Imidazobenzodiazepine der Formel (I) nach Anspruch 1, **dadurch gekennzeichnet, daß** in der Formel (I) $R_1$ für eine Cyclopropylgruppe steht, und $R_2$, $R_3$, X und Y wie vorstehend definiert sind, sowie ihre Additionssalze mit mineralischen oder organischen Säuren.

3. Neue Imidazobenzodiazepine der Formel (I) nach Anspruch 1 oder 2, **dadurch gekennzeichnet, daß** in der Formel (I) $R_1$ für eine Cyclopropylgruppe steht, $R_2$ und $R_3$, die gleich oder verschieden sind, für ein Wasserstoffatom, eine Methylgruppe stehen, X und Y, die gleich oder verschieden sind, für ein Wasserstoffatom, ein Fluor- oder Bromatom stehen, sowie ihre Additionssalze mit mineralischen oder organischen Säuren.

4. 3-(5,6-Dihydro-5-methyl-6-oxo-4H-imidazo [1,5a][1,4]benzodiazepinyl)cyclopropylmethanon.

5. Verfahren zur Herstellung der neuen Imidazobenzodiazepine der allgemeinen Formel ($I_A$) gemäß Anspruch 1

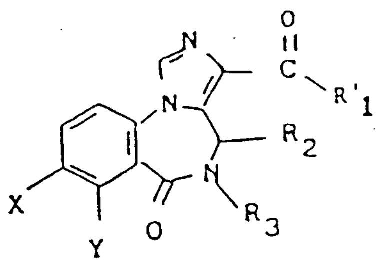

$(I_A)$

worin $R_2$, $R_3$, X und Y wie vorstehend definiert sind, und $R'_1$ für einen Phenylrest, einen Cycloalkylrest mit 4 bis 6 Kohlenstoffatomen oder eine Gruppe der Formel (II), worin $R_4$ für ein Wasserstoffatom steht, und $R_5$ und $R_6$, die gleich oder verschieden sind, für ein Wasserstoffatom oder einen Alkylrest mit 1 bis 5 Kohlenstoffatomen stehen, steht, sowie ihre Salze, **dadurch gekennzeichnet, daß** man

-- entweder eine Verbindung der Formel (V):

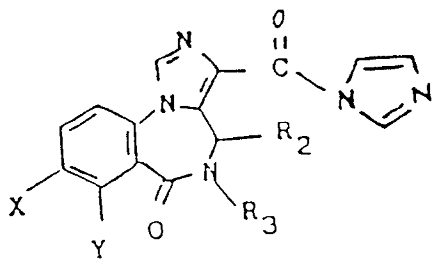

(V)

worin X,Y, $R_2$ und $R_3$ wie vorstehend definiert sind, mit einer Verbindung der Formel (VI):

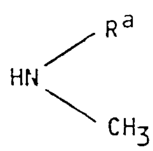

(VI)

worin $R^a$ für eine Methyl- oder Methoxygruppe steht, oder ein Säureadditionssalz umsetzt, um eine Verbindung der Formel (III):

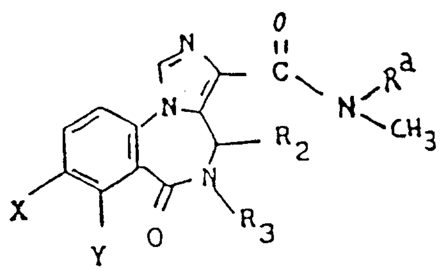

(III)

worin $R_2$, $R_3$, $R^a$, X und Y wie vorstehend definiert sind, zu erhalten, dann die Verbindung der Formel III mit einer Verbindung der Formel (IV):

$M\text{-}R'_1$ (IV),

worin M für ein Alkalimetallatom, wie ein Lithiumatom, oder eine -Mg-Hal-Gruppe, worin Hal für ein Chlor-, Brom- oder Iodatom steht, steht, und $R'_1$ wie vorstehend definiert sind, umsetzt, um die gewünschte Verbindung der Formel ($I_A$) zu erhalten, die gegebenenfalls in ein Salz überführt werden kann,

-- oder eine Verbindung der Formel (VIII):

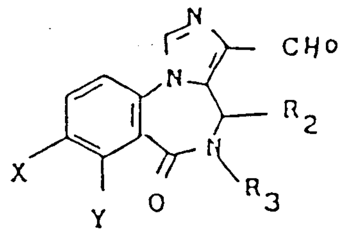

(VIII)

worin X,Y, $R_2$ und $R_3$ wie vorstehend definiert sind, mit einer Verbindung der vorstehend definierten Formel (IV) umsetzt, um eine Verbindung der Formel (VII):

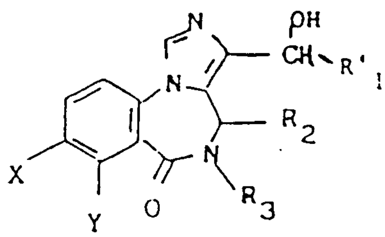

(VII)

worin $R'_1$, $R_2$, $R_3$, X und Y wie vorstehend definiert sind, zu erhalten, anschließend die so erhaltene Verbindung der Formel (VII) oxidiert, um die gewünschte Verbindung der Formel ($I_A$) zu erhalten, die gegebenenfalls in ein Salz überführt werden kann.

**6.** Verfahren zur Herstellung nach Anspruch 5, **dadurch gekennzeichnet, daß**
-- die Reaktion der Verbindung der Formel (V) mit der Verbindung der Formel (VI) bevorzugt in Dimethylformamid durchgeführt wird;
-- die Reaktion der Verbindung der Formel (III) oder (VIII) mit der Verbindung der Formel (IV) unter wasserfreien Bedingungen in einem organischen Lösungsmittel, wie Tetrahydrofuran, durchgeführt wird;
-- die Oxidation der Verbindung der Formel (VII) bevorzugt mit Mangandioxid, Salpetersäure, Eisen-(III)-chlorid oder Chromoxid in Gegenwart von Pyridin oder nach dem Oppenauerverfahren oder schließlich durch Dehydrierung in Gegenwart eines Katalysators auf Kupferbasis durchgeführt wird.

**7.** Verfahren zur Herstellung der neuen Imidazobenzodiazepine der Formel (I) gemäß Anspruch 1, der allgemeinen Formel ($I_B$):

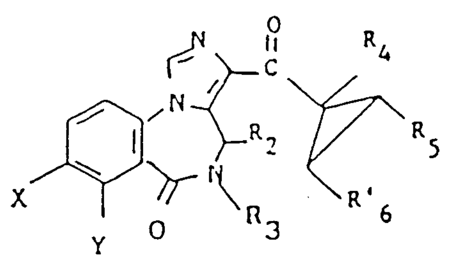

($I_B$)

worin $R_2$, $R_3$, $R_4$, $R_5$, X und Y wie vorstehend definiert sind, und $R'_6$ für ein Wasserstoffatom, ein Halogenatom, einen Alkylrest mit 1 bis 5 Kohlenstoffatomen oder einen Alkoxycarbonylrest mit 2 bis 5 Kohlenstoffatomen, einen Phenylrest, eine Cyanogruppe oder eine Gruppe

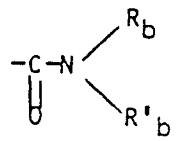

worin $R_b$ und $R'_b$ gleich oder verschieden sind und für ein Wasserstoffatom oder einen Alkylrest mit 1 bis 5 Kohlenstoffatomen stehen, steht, **dadurch gekennzeichnet, daß** man

-- entweder eine Verbindung der Formel (III), wie vorstehend definiert, mit einer Verbindung der Formel (X):

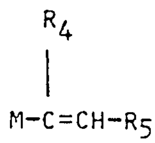

(X)

worin M, $R_4$ und $R_5$ wie vorstehend definiert sind, umsetzt, um eine Verbindung der Formel (IX):

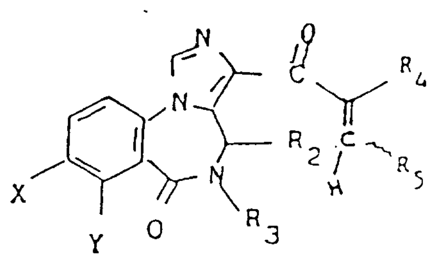

(IX)

zu erhalten, worin $R_2$, $R_3$, $R_4$, $R_5$, X und Y wie vorstehend definiert sind,

-- oder eine Verbindung der vorstehend definierten Formel (VIII) mit einer Verbindung der Formel (X) umsetzt, um eine Verbindung der Formel (XI):

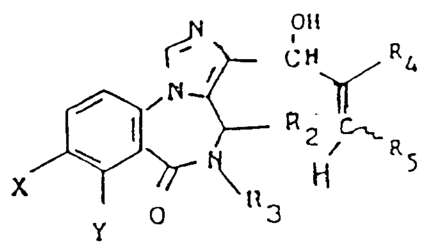

(XI)

worin $R_2$, $R_3$, $R_4$, $R_5$, X und Y wie vorstehend definiert sind, zu erhalten, anschließend die so erhaltene Verbindung der Formel (XI) mit einem Oxidationsmittel oxidiert, um eine Verbindung der Formel (IX) zu erhalten, die man mit einem geeigneten Cyclisierungsmittel umsetzt, um eine Verbindung der vorstehend definierten Formel ($I_B$), worin $R'_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen oder einen Phenylrest steht, zu erhalten, anschließend gegebenenfalls die erhaltene Verbindung, worin $R'_6$ für einen Alkoxycarbonylrest steht, verseift, um eine Verbindung der Formel (XIII):

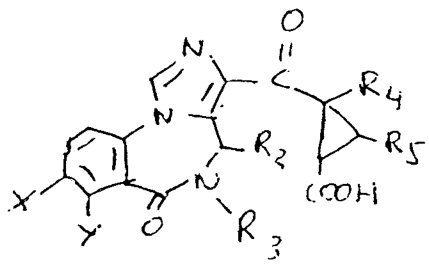

(XIII)

worin $R_2$, $R_3$, $R_4$, $R_5$, X und Y wie vorstehend definiert sind, zu erhalten, anschließend die Verbindung der Formel (XIII) mit einem Amin der Formel (XII):

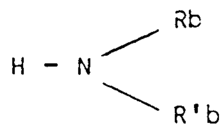

(XII)

worin $R_b$ und $R'_b$ wie vorstehend definiert sind, umsetzt, um die Verbindung der Formel ($I_B$), worin $R'_6$ für eine Gruppe der Formel

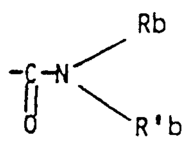

steht, zu erhalten, anschließend gegebenenfalls die zuletztgenannte Verbindung, worin $R_b$ und $R'_b$ für ein Wasserstoffatom stehen, dehydratisiert, um eine Verbindung der Formel $I_B$, worin $R'_6$ für eine Cyanogruppe steht, zu erhalten, und gegebenenfalls die so erhaltenen Verbindungen der Formel $I_B$ in ein Salz überführt.

**8.** Verfahren zur Herstellung nach Anspruch 7, **dadurch gekennzeichnet, daß** die Umsetzung der Verbindungen der Formeln (III) und (VIII) mit der Verbindung der Formel (X) unter wasserfreien Bedingungen in einem organischen Lösungsmittel, wie Tetrahydrofuran, durchgeführt wird.

**9.** Verfahren zur Herstellung nach Anspruch 7, **dadurch gekennzeichnet, daß** die Oxidation der Verbindung der Formel (XI) bevorzugt mit Mangandioxid, Salpetersäure, Eisen(III)-chlorid oder Chromoxid in Gegenwart von Pyridin, oder auch nach dem Oppenauerverfahren oder auch durch Dehydrieren in Gegenwart eines Katalysators auf Kupferbasis durchgeführt wird.

**10.** Verfahren zur Herstellung nach Anspruch 7, **dadurch gekennzeichnet, daß**

- das geeignete Cyclisierungsmittel ein Reagenz mit der Fähigkeit zur Einführung der

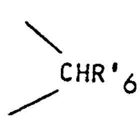

-Gruppe in die Doppelbindung ist;
- wenn eine Verbindung der Formel ($I_B$), worin $R_4$ für einen Alkylrest mit 1 bis 5 Kohlenstoffatomen steht, $R_5$ für ein Wasserstoffatom steht, und $R'_6$ für ein Wasserstoffatom oder einen Alkylrest mit 1 bis 5 Kohlenstoffatomen steht, erhalten werden soll, die Cyclisierung bevorzugt mittels Trialkyloxosulfoniumiodid in einem organischen Lösungsmittel, wie Dimethylformamid, durchgeführt wird;

- wenn eine Verbindung der Formel ($I_B$), worin $R_4$ für ein Wasserstoffatom steht, $R_5$ für einen Alkylrest mit 1 bis 5 Kohlenstoffatomen steht, und $R'_6$ für ein Wasserstoffatom oder einen Alkylrest mit 1 bis 5 Kohlenstoffatomen steht, erhalten werden soll, die Cyclisierung bevorzugt mittels Dimethylaminoalkylphenyloxosulfoniumtetrafluorborat in einem organischen Lösungsmittel, wie Dimethylformamid, durchgeführt wird;
- wenn eine Verbindung der Formel ($I_B$), worin $R_4$ für ein Wasserstoffatom steht, und $R'_6$ für einen Alkoxycarbonyl- oder -phenylrest steht, erhalten werden soll, die Cyclisierung bevorzugt mittels Dimethylsulfuranylidenacetat oder des Benzyldimethylsulfoniumanionsin einem organischen Lösungsmittel, wie Chloroform, durchgeführt wird;
- wenn eine Verbindung der Formel ($I_B$), worin $R_4$ für ein Wasserstoffatom steht, und $R'_6$ für ein Halogenatom steht, erhalten werden soll, die Cyclisierung bevorzugt mittels Dimethylaminophenyloxosulfoniumhalogenmethylylid in einem organischen Lösungsmittel, wie Dimethylformamid, durchgeführt wird;

**11.** Verfahren zur Herstellung nach Anspruch 7, **dadurch gekennzeichnet, daß**
- die Verseifung der Verbindung der Formel ($I_B$), worin $R'_6$ für einen Alkoxycarbonylrest steht, bevorzugt mittels eines Alkalihydroxids, wie Natriumhydroxid, durchgeführt wird;
- die Umsetzung der Verbindung der Formel ($I_B$) mit dem Amin der Formel (XII) bevorzugt in einem wasserfreien organischen Lösungsmittel in Gegenwart von Carbonyldiimidazol durchgeführt wird;
- die Dehydratisierung der Verbindung der Formel ($I_B$) bevorzugt mit einem starken Säureanhydrid, wie Trifluoressigsäureanhydrid, in einem organischen Lösungsmittel, wie Dichlormethan, durchgeführt wird.

**12.** Medikamente, **dadurch gekennzeichnet, daß** sie die neuen Imidazobenzodiazepine der Formel (I) nach Anspruch 1, sowie ihre pharmazeutisch verträglichen Salze enthalten.

**13.** Medikamente, **dadurch gekennzeichnet, daß** sie die neuen Imidazobenzodiazepine nach einem der Ansprüche 2, 3 oder 4, sowie ihre pharmazeutisch verträglichen Salze enthalten.

**14.** Pharmazeutische Präparate, **dadurch gekennzeichnet, daß** sie als Wirkstoff mindestens eines der Medikamente nach einem der Ansprüche 12 oder 13 enthalten.

**15.** Als neue industriell verwendbare Produkte:
- Die Verbindungen der Formel (VII):

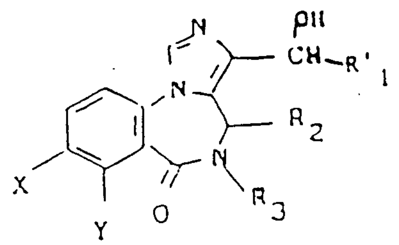

(VII)

worin $R'_1$, $R_2$, $R_3$, X und Y wie vorstehend definiert sind;
- Die Verbindungen der Formel (XI):

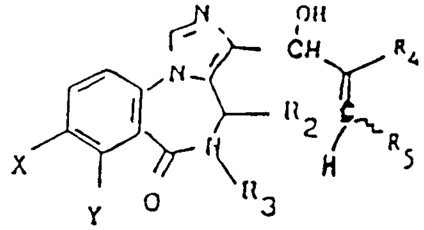

(XI)

worin $R_2$, $R_3$, $R_4$, $R_5$, X und Y wie vorstehend definiert sind;
- Die Verbindungen der Formel (IX):

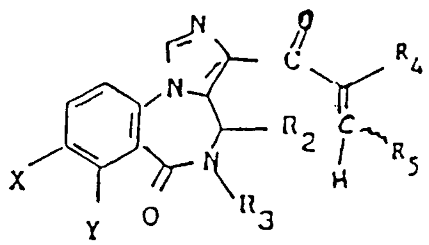

(IX)

worin $R_2$, $R_3$, $R_4$, $R_5$ wie vorstehend definiert sind;
- Die Verbindungen der Formel (III):

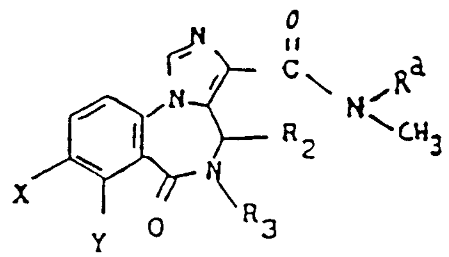

(III)

worin $R_2$, $R_3$, $R^a$, X und Y wie vorstehend definiert sind;
- Die Verbindungen der Formel (XIII):

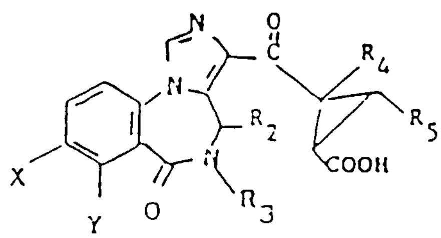

(XIII)

worin $R_2$, $R_3$, $R_4$, $R_5$, X und Y wie vorstehend definiert sind.

**Patentansprüche für folgenden Vertragsstaat : ES**

**1.** Verfahren zur Herstellung neuer Imidazobenzodiazepine der allgemeinen Formel (I)

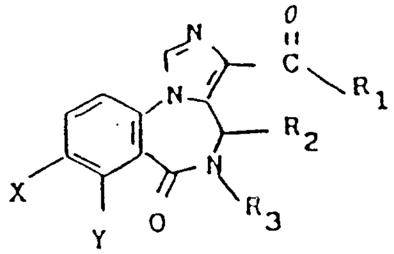

(I)

worin $R_1$ für einen Phenylrest, einen Cycloalkylrest mit 4 bis 6 Kohlenstoffatomen oder eine Gruppe der Formel (II):

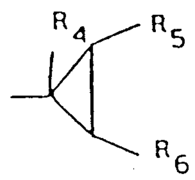

(II)

worin $R_4$ für ein Wasserstoffatom oder einen linearen oder verzweigten Alkylrest mit 1 bis 5 Kohlenstoffatomen steht, $R_5$ für ein Wasserstoffatom oder einen linearen oder verzweigten Alkylrest mit 1 bis 5 Kohlenstoffatomen steht, $R_6$ für ein Wasserstoffatom, einen linearen oder verzweigten Alkylrest mit 1 bis 5 Kohlenstoffatomen, einen Phenylrest, ein Halogenatom, einen Alkoxycarbonylrest mit 2 bis 5 Kohlenstoffatomen, eine Cyanogruppe, eine Amidogruppe, eine Mono- oder Dialkylamidogruppe, deren lineare oder verzweigte Alkylgruppen 1 bis 5 Kohlenstoffatome umfassen, steht, steht, $R_2$ und $R_3$, die gleich oder verschieden sind, für ein Wasserstoffatom, einen linearen oder verzweigten Alkylrest mit 1 bis 5 Kohlenstoffatomen, einen Cycloalkylrest mit 3 bis 5 Kohlenstoffatomen stehen, oder $R_2$ und $R_3$ zusammen einen Alkylenrest mit 3 bis 5 Kohlenstoffatomen bilden, X und Y, die gleich oder verschieden sind, für ein Wasserstoffatom, ein Halogenatom, einen Alkyl- oder Alkoxyrest mit 1 bis 3 Kohlenstoffatomen, eine Nitro-, Azido-, Nitril- oder Trifluormethylgruppe stehen, sowie ihre Additionssalze mit mineralischen oder organischen Säuren, **dadurch gekennzeichnet, daß** man

a) zur Herstellung von Verbindungen der Formel ($I_A$):

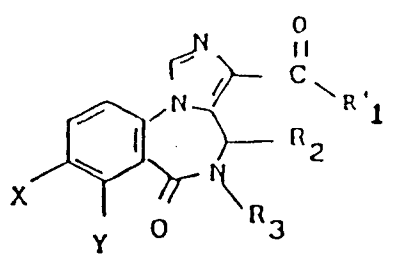

($I_A$)

worin $R_2$, $R_3$, X und Y wie vorstehend definiert sind und $R'_1$ für einen Phenylrest, einen Cycloalkylrest mit 4 bis 6 Kohlenstoffatomen oder eine Gruppe der Formel (II), worin $R_4$ für ein Wasserstoffatom steht, und $R_5$ und $R_6$, die gleich oder verschieden sind, für ein Wasserstoffatom oder einen Alkylrest mit 1 bis 5 Kohlenstoffatomen stehen, steht oder ihrer Salze,

-- entweder eine Verbindung der Formel (V):

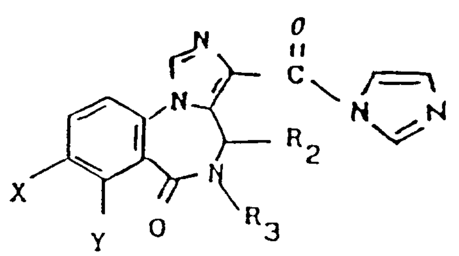

(V)

worin X,Y, $R_2$ und $R_3$ wie vorstehend definiert sind, mit einer Verbindung der Formel (VI):

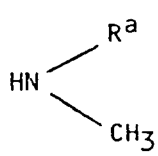

(VI)

worin $R^a$ für eine Methyl- oder Methoxygruppe steht, oder ein Säureadditionssalz umsetzt, um

eine Verbindung der Formel (III):

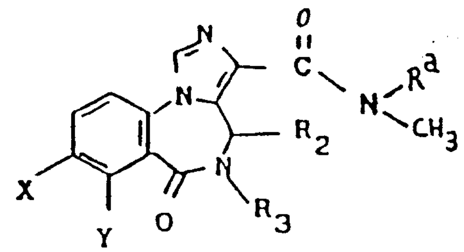

(III)

worin $R_2$, $R_3$, $R^a$, X und Y wie vorstehend definiert sind, zu erhalten, dann die Verbindung der Formel (III) mit einer Verbindung der Formel (IV):

M-$R'_1$ (IV),

worin M für ein Alkalimetallatom, wie ein Lithiumatom, oder eine -Mg-Hal-Gruppe, worin Hal für ein Chlor- ,Brom- oder Iodatom steht, steht und $R'_1$ wie vorstehend definiert sind, umsetzt, um die gewünschte Verbindung der Formel ($I_A$) zu erhalten, die gegebenenfalls in ein Salz überführt werden kann,
-- oder eine Verbindung der Formel (VIII):

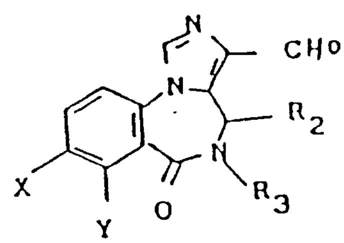

(VIII)

worin X,Y, $R_2$ und $R_3$ wie vorstehend definiert sind, mit einer Verbindung der vorstehend definierten Formel (IV) umsetzt, um eine Verbindung der Formel (VII):

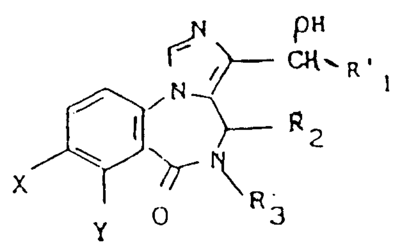

(VII)

worin $R'_1$, $R_2$, $R_3$, X und Y wie vorstehend definiert sind, zu erhalten, anschließend die so erhaltene Verbindung der Formel (VII) oxidiert, um die gewünschte Verbindung der Formel ($I_A$) zu erhalten, die gegebenenfalls in ein Salz überführt werden kann,

b) zu Herstellung von Verbindungen der Formel ($I_B$):

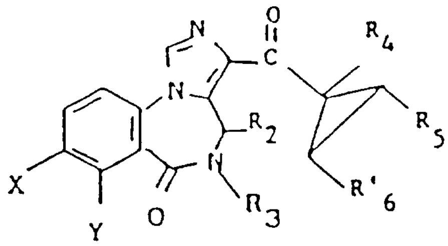

($I_B$)

worin $R_2$, $R_3$, $R_4$, $R_5$, X und Y wie vorstehend definiert sind und $R'_6$ für ein Wasserstoffatom, ein Halogenatom, einen Alkylrest mit 1 bis 5 Kohlenstoffatomen oder einen Alkoxycarbonylrest mit 2 bis 5 Kohlenstoffatomen, einen Phenylrest, eine Cyanogruppe oder eine Gruppe

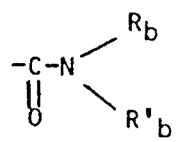

,

worin $R_b$ und $R'_b$ gleich oder verschieden sind und für ein Wasserstoffatom oder einen Alkylrest mit 1 bis 5 Kohlenstoffatomen stehen, steht,

-- man entweder eine Verbindung der Formel (III), wie vorstehend definiert, mit einer Verbindung der Formel (X):

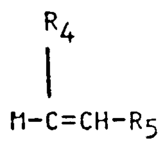

(X)

worin M, $R_4$ und $R_5$ wie vorstehend definiert sind, umsetzt, um eine Verbindung der Formel (IX):

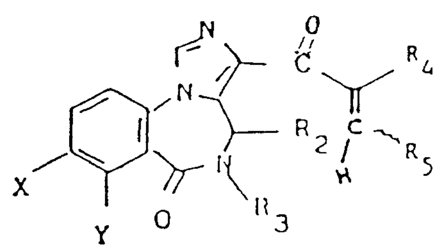

(IX)

zu erhalten, worin $R_2$, $R_3$, $R_4$, $R_5$, X und Y wie vorstehend definiert sind,

-- oder eine Verbindung der vorstehend definierten Formel (VIII) mit einer Verbindung der Formel (X) umsetzt, um eine Verbindung der Formel (XI):

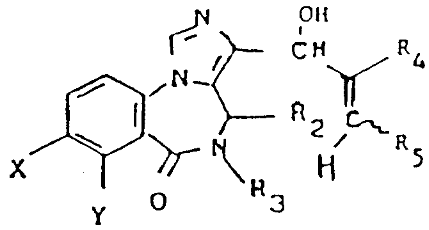

(XI)

worin $R_2$, $R_3$, $R_4$, $R_5$, X und Y wie vorstehend definiert sind, zu erhalten, anschließend die so erhaltene Verbindung der Formel (XI) mit einem Oxidationsmittel oxidiert, um eine Verbindung der Formel (IX) zu erhalten, die man mit einem geeigneten Cyclisierungsmittel umsetzt, um eine Verbindung der vorstehend definierten Formel ($I_B$), worin $R'_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen oder einen Phenylrest steht, zu erhalten, anschließend gegebenenfalls die erhaltene Verbindung, worin $R'_6$ für einen Alkoxycarbonylrest steht, verseift, um eine Verbindung der Formel (XIII):

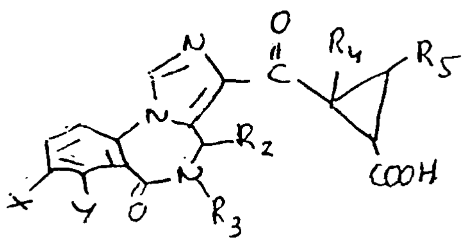

(XIII)

worin $R_2$, $R_3$, $R_4$, $R_5$, X und Y wie vorstehend definiert sind, zu erhalten, anschließend die Verbindung der Formel (XIII) mit einem Amin der Formel (XII)

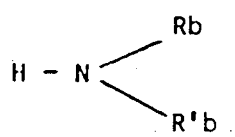

(XII)

worin $R_b$ und $R'_b$ wie vorstehend definiert sind, umsetzt, um die Verbindung der Formel ($I_B$), worin $R'_6$ für eine Gruppe der Formel

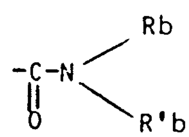

steht, zu erhalten, anschließend gegebenenfalls die zuletztgenannte Verbindung, worin $R_b$ und $R'_b$ für ein Wasserstoffatom stehen, dehydratisiert, um eine Verbindung der Formel ($I_B$),worin $R'_6$ für eine Cyanogruppe steht, zu erhalten, und gegebenenfalls die so erhaltenen Verbindungen der Formel ($I_B$) in ein Salz überführt.

2. Verfahren nach Anspruch 1, **dadurch gekennzeichnet, daß** man eine Verbindung der Formel (IV), worin $R'_1$ für einen Cyclopropylrest steht, oder eine Verbindung der Formel (X), worin $R_4$ und $R_5$ für ein Wasserstoffatom stehen, verwendet.

3. Verfahren nach Anspruch 1 oder 2, **dadurch gekennzeichnet, daß** man eine Verbindung der Formel (V) oder (VIII), worin $R_2$ und $R_3$, die gleich oder verschieden sind, für ein Wasserstoffatom, einen

Methylrest stehen, X und Y, die gleich oder verschieden sind, für ein Wasserstoffatom, ein Fluor- oder Bromatom stehen, verwendet.

**4.** Verfahren nach Anspruch 1 oder 2, **dadurch gekennzeichnet, daß** man eine Verbindung der Formel (V) oder (VIII), worin $R_3$ für einen Methylrest steht, $R_2$ für ein Wasserstoffatom steht, X und Y für ein Wasserstoffatom stehen, und eine Verbindung der Formel (IV), worin $R'_1$ für einen Cyclopropylrest steht, oder eine Verbindung der Formel (X), worin $R_4$ und $R_5$ für ein Wasserstoffatom stehen, verwendet.

**Patentansprüche für folgenden Vertragsstaat : GR**

**1.** Verfahren zur Herstellung neuer Imidazobenzodiazepine der allgemeinen Formel (I)

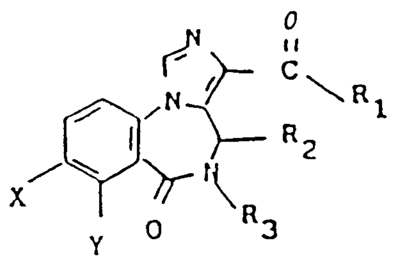

(I)

worin $R_1$ für einen Phenylrest, einen Cycloalkylrest mit 4 bis 6 Kohlenstoffatomen oder eine Gruppe der Formel (II):

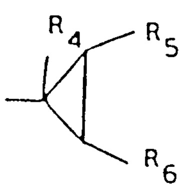

(II)

worin $R_4$ für ein Wasserstoffatom oder einen linearen oder verzweigten Alkylrest mit 1 bis 5 Kohlenstoffatomen steht, $R_5$ für ein Wasserstoffatom oder einen linearen oder verzweigten Alkylrest mit 1 bis 5 Kohlenstoffatomen steht, $R_6$ für ein Wasserstoffatom, einen linearen oder verzweigten Alkylrest mit 1 bis 5 Kohlenstoffatomen, einen Phenylrest, ein Halogenatom, einen Alkoxycarbonylrest mit 2 bis 5 Kohlenstoffatomen, eine Cyanogruppe, eine Amidogruppe, eine Mono- oder Dialkylamidogruppe, deren lineare oder verzweigte Alkylgruppen 1 bis 5 Kohlenstoffatome umfassen steht, steht, $R_2$ und $R_3$, die gleich oder verschieden sind, für ein Wasserstoffatom, einen linearen oder verzweigten Alkylrest mit 1 bis 5 Kohlenstoffatomen, einen Cycloalkylrest mit 3 bis 5 Kohlenstoffatomen stehen, oder $R_2$ und $R_3$ zusammen einen Alkylenrest mit 3 bis 5 Kohlenstoffatomen bilden, X und Y, die gleich oder verschieden sind, für ein Wasserstoffatom, ein Halogenatom, einen Alkyl- oder Alkoxyrest mit 1 bis 3 Kohlenstoffatomen, eine Nitro-, Azido-, Nitril- oder Trifluormethylgruppe stehen, sowie ihre Additionssalze mit mineralischen oder organischen Sauren, **dadurch gekennzeichnet, daß** man

a) zur Herstellung von Verbindungen der Formel ($I_A$):

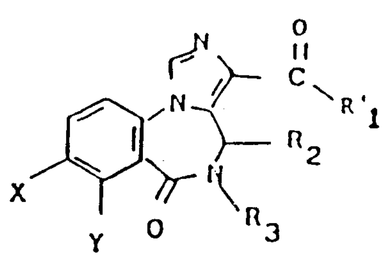

($I_A$)

worin $R_2$, $R_3$, X und Y wie vorstehend definiert sind und $R'_1$ für einen Phenylrest, einen Cycloalkylrest mit 4 bis 6 Kohlenstoffatomen oder eine Gruppe der Formel (II), worin $R_4$ für ein Wasserstoffatom steht, und $R_5$ und $R_6$, die gleich oder verschieden sind, für ein Wasserstoffatom oder einen Alkylrest mit 1 bis 5 Kohlenstoffatomen stehen, steht, oder ihrer Salze,

-- entweder eine Verbindung der Formel (V):

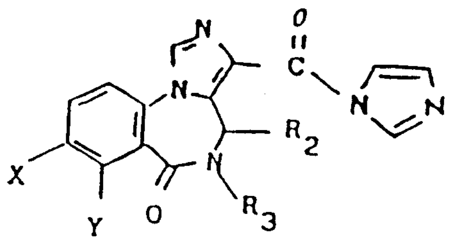

(V)

worin X,Y, $R_2$ und $R_3$ wie vorstehend definiert sind, mit einer Verbindung der Formel (VI):

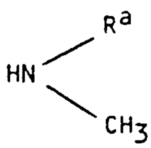

(VI)

worin $R^a$ für eine Methyl- oder Methoxygruppe steht, oder ein Säureadditionssalz umsetzt, um eine Verbindung der Formel (III):

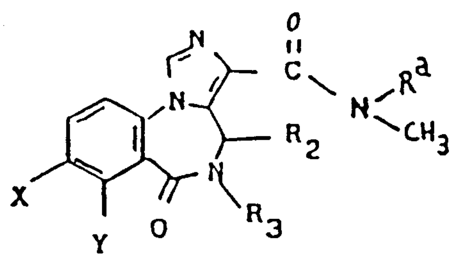

(III)

worin $R_2$, $R_3$, $R^a$, X und Y wie vorstehend definiert sind, zu erhalten, dann die Verbindung der Formel (III) mit einer Verbindung der Formel (IV)

M-$R'_1$ (IV),

worin M für ein Alkalimetallatom, wie ein Lithiumatom, oder eine -Mg-Hal-Gruppe, worin Hal für ein Chlor- ,Brom- oder Iodatom steht, steht und $R'_1$ wie vorstehend definiert sind, umsetzt, um die gewünschte Verbindung der Formel ($I_A$) zu erhalten, die gegebenenfalls in ein Salz überführt werden kann,

-- oder eine Verbindung der Formel (VIII):

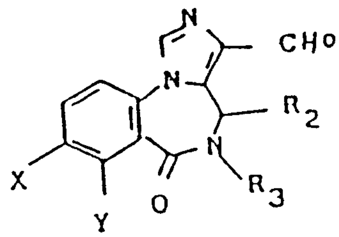

(VIII)

worin X,Y, $R_2$ und $R_3$ wie vorstehend definiert sind, mit einer Verbindung der vorstehend definierten Formel (IV) umsetzt, um eine Verbindung der Formel (VII):

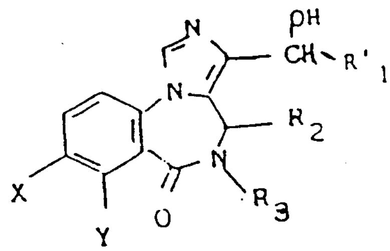

(VII)

worin $R'_1$, $R_2$, $R_3$, X und Y wie vorstehend definiert sind, zu erhalten, anschließend die so erhaltene Verbindung der Formel (VII) oxidiert, um die gewünschte Verbindung der Formel ($I_A$) zu erhalten, die gegebenenfalls in ein Salz überführt werden kann,

b) zur Herstellung von Verbindungen der Formel ($I_B$):

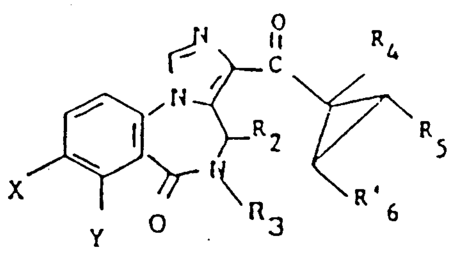

($I_B$)

worin $R_2$, $R_3$, $R_4$, $R_5$, X und Y wie vorstehend definiert sind, und $R'_6$ für ein Wasserstoffatom, ein Halogenatom, einen Alkylrest mit 1 bis 5 Kohlenstoffatomen oder einen Alkoxycarbonylrest mit 2 bis 5 Kohlenstoffatomen, einen Phenylrest, eine Cyanogruppe oder eine Gruppe

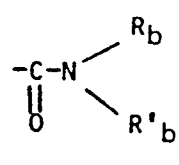

,

worin $R_b$ und $R'_b$ gleich oder verschieden sind und für ein Wasserstoffatom oder einen Alkylrest mit 1 bis 5 Kohlenstoffatomen stehen, steht,

-- man entweder eine Verbindung der Formel (III), wie vorstehend definiert, mit einer Verbindung der Formel (X):

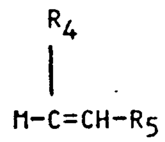

(X)

worin M, $R_4$ und $R_5$ wie vorstehend definiert sind, umsetzt, um eine Verbindung der Formel (IX):

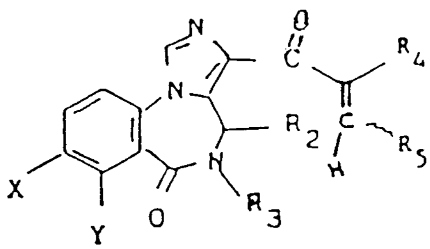

(IX)

zu erhalten, worin $R_2$, $R_3$, $R_4$, $R_5$, X und Y wie vorstehend definiert sind,
-- oder eine Verbindung der vorstehend definierten Formel (VIII) mit einer Verbindung der Formel X umsetzt, um eine Verbindung der Formel (XI):

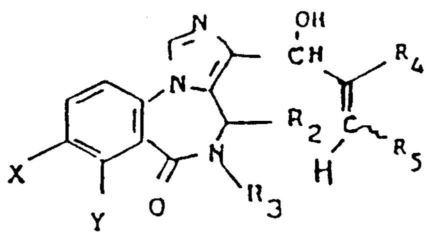

(XI)

worin $R_2$, $R_3$, $R_4$, $R_5$, X und Y wie vorstehend definiert sind, zu erhalten, anschließend die so erhaltene Verbindung der Formel (XI) mit einem Oxidationsmittel oxidiert, um eine Verbindung der Formel (IX) zu erhalten, die man mit einem geeigneten Cyclisierungsmittel umsetzt, um eine Verbindung der vorstehend definierten Formel ($I_B$), worin $R'_6$ für ein Wasserstoffatom, ein Halogenatom, einen Alkylrest mit 1 bis 5 Kohelnstoffatomen, einen Alkoxycarbonylrest mit 2 bis 5 Kohlenstoffatomen oder einen Phenylrest steht, zu erhalten, anschließend gegebenenfalls die erhaltene Verbindung, worin $R'_6$ für einen Alkoxycarbonylrest steht, verseift, um eine Verbindung der Formel (XIII):

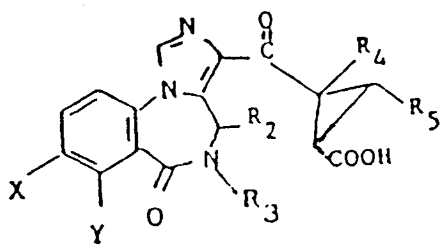

(XIII)

worin $R_2$, $R_3$, $R_4$, $R_5$, X und Y wie vorstehend definiert sind, zu erhalten, anschließend die Verbindung der Formel (XIII) mit einem Amin der Formel (XII):

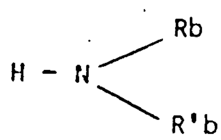

(XII)

worin $R_b$ und $R'_b$ wie vorstehend definiert sind, umsetzt, um die Verbindung der Formel ($I_B$), worin $R'_6$ für eine Gruppe der Formel

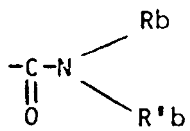

steht, zu erhalten, anschließend gegebenenfalls die zuletztgenannte Verbindung, worin $R_b$ und $R'_b$ für ein Wasserstoffatom stehen dehydratisiert, um eine Verbindung der Formel ($I_B$), worin $R'_6$ für eine Cyanogruppe steht, zu erhalten, und gegebenenfalls die so erhaltenen Verbindungen der Formel $I_B$ in ein Salz überführt.

2. Verfahren nach Anspruch 1, **dadurch gekennzeichnet, daß** man eine Verbindung der Formel (IV), worin $R'_1$ für einen Cyclopropylrest steht, oder eine Verbindung der Formel (X), worin $R_4$ und $R_5$ für ein Wasserstoffatom stehen, verwendet.

3. Verfahren nach Anspruch 1 oder 2, **dadurch gekennzeichnet, daß** man eine Verbindung der Formel (V) oder (VIII), worin $R_2$ und $R_3$, die gleich oder verschieden sind, für ein Wasserstoffatom, einen Methylrest stehen, X und Y, die gleich oder verschieden sind, für ein Wasserstoffatom, ein Fluor- oder Bromatom stehen, verwendet.

4. Verfahren nach Anspruch 1 oder 2, **dadurch gekennzeichnet, daß** man eine Verbindung der Formel (V) oder (VIII), worin $R_3$ für einen Methylrest steht, $R_2$ für ein Wasserstoffatom steht, X und Y für ein Wasserstoffatom stehen, und eine Verbindung der Formel (IV), worin $R'_1$ für einen Cyclopropylrest steht, oder eine Verbindung der Formel (X), worin $R_4$ und $R_5$ für ein Wasserstoffatom stehen, verwendet.

5. Verbindungen der Formel (VII), (XI), (IX), (III) und (XIII) nach Anspruch 1 als neue Produkte mit industrieller Verwendung.

6. Verfahren zur Herstellung von pharmazeutischen Präparaten, **dadurch gekennzeichnet, daß** man als Wirkstoff mindestens eine der Verbindungen der Formel (I) nach Anspruch 1 oder mindestens eines ihrer Additionssalze mit pharmazeutisch verträglichen, mineralischen oder organischen Säuren in einer für diese Verwendung geeigneten Form verwendet.